(19) Europäisches Patentamt
European Patent Office
Office européen des brevets

(11) Veröffentlichungsnummer: **0 224 094 A2**

(12) **EUROPÄISCHE PATENTANMELDUNG**

(21) Anmeldenummer: 86115457.3

(22) Anmeldetag: 07.11.86

(51) Int. Cl.[4]: C07D 231/16 , C07D 231/14 , C07D 401/04 , C07F 3/14 , A01N 43/56 , A01N 43/40 , A01N 55/06

(30) Priorität: 18.11.85 DE 3540839

(43) Veröffentlichungstag der Anmeldung: 03.06.87 Patentblatt 87/23

(84) Benannte Vertragsstaaten: AT BE CH DE ES FR GB IT LI NL SE

(71) Anmelder: BAYER AG
Konzernverwaltung RP Patentabteilung
D-5090 Leverkusen 1 Bayerwerk(DE)

(72) Erfinder: Jensen-Korte, Uta, Dr.
Geibelstrasse 9
D-4000 Düsseldorf 1(DE)
Erfinder: Gehring, Reinhold, Dr.
Dasnöckel 49
D-5600 Wuppertal 11(DE)
Erfinder: Stetter, Jörg, Dr.
Gellertweg 4
D-5600 Wuppertal 1(DE)
Erfinder: Schallner, Otto, Dr.
Noldeweg 22
D-4019 Monheim(DE)
Erfinder: Santel, Hans-Joachim, Dr.
Grünstrasse 9a
D-5090 Leverkusen 1(DE)
Erfinder: Schmidt, Robert R., Dr.
Im Waldwinkel 110
D-5060 Bergisch Gladbach 2(DE)

(54) **1-Aryl-pyrazole.**

(57) Die Erfindung betrifft 1-Aryl-pyrazole der Formel (I),

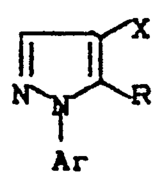

(I)

in welcher
X für Cyano oder Nitro steht,
R für Cyano oder für einen Rest -CO-Y-R[1] steht,
wobei die Definition von Y und R[1] und Ar der Beschreibung zu entnehmen ist,
mehrere Verfahren zu ihrer Herstellung und ihre Verwendung als Herbizide.

EP 0 224 094 A2

## 1-Aryl-pyrazole

Die Erfindung betrifft neue 1-Aryl-pyrazole, mehrere Verfahren zu ihrer Herstellung und ihre Verwendung als Herbizide.

Es ist bereits bekannt, daß bestimmte 1-Aryl-pyrazole wie beispielsweise das 4-Cyano-5-propionamido-1-(2,4,6-trichlorphenyl)-pyrazol herbizide Eigenschaften besitzen (vgl. z.B. DE-OS 3 226 513).

Die herbizide Wirksamkeit dieser vorbekannten Verbindungen gegenüber Unkräutern ist jedoch ebenso wie ihre Verträglichkeit gegenüber wichtigen Kulturpflanzen nicht immer in allen Anwendungsgebieten völlig zufriedenstellend.

Weiterhin sind einige 1-Aryl-pyrazole wie beispielsweise das 4,5-Dicyano-1-phenyl-pyrazol, das 5-Carboxy-4-nitro-1-(3,4-dichlorphenyl)-pyrazol, das 5-Carboxy-4-nitro-1-phenylpyrazol, das 5-Carboxy-4-nitro-1-p-tolylpyrazol und das 5-Carboxy-4-nitro-1-(3-nitrophenyl)-pyrazol als Zwischenprodukte bekannt (vgl. Rev. Latinoam Quim. 13, 100-102 [1982]; DE-P 1 229 095).

Über eine herbizide Wirksamkeit dieser vorbekannten Verbindungen ist jedoch nichts bekannt.

Es wurden neue 1-Aryl-pyrazole der allgemeinen Formel (I),

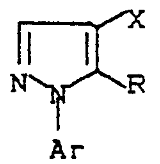 (I)

in welcher

X für Cyano oder Nitro steht,

R für Cyano oder für einen Rest $-CO-Y-R^1$ steht,

wobei

Y für Sauerstoff, Schwefel oder einen Rest $-\underset{R^2}{\underset{|}{N}}-$ steht,

$R^1$ für Wasserstoff, für Alkyl, Hydroxyalkyl, Alkoxyalkyl, Alkylthioalkyl, Halogenalkyl, Alkenyl, Halogenalkenyl, Alkinyl oder für jeweils gegebenenfalls substituiertes Cycloalkyl, Aralkyl oder Aryl steht, für einen Rest

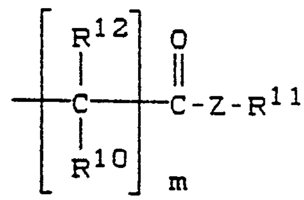

steht,

oder $R^1$ für den Fall, daß Y für Sauerstoff, Schwefel oder einen Rest $-\underset{|}{N}-SO_2-R^3$ steht,

auch für ein salzartig gebundenes anorganisches oder organisches Kation steht,

$R^2$ für Wasserstoff, für Alkyl, Hydroxyalkyl, Alkoxyalkyl, Alkylthioalkyl, Halogenalkyl, Alkenyl, Alkinyl oder Halogenalkenyl, für jeweils gegebenenfalls substituiertes Cycloalkyl, Aralkyl oder Aryl steht oder für einen Rest $-SO_2-R^3$ steht, wobei

$R^3$ für jeweils gegebenenfalls substituiertes Alkyl, Aralkyl oder Aryl steht,

$R^{10}$ und $R^{12}$ unabhängig voneinander für Wasserstoff oder Alkyl stehen,

$R^{11}$ für Wasserstoff, Alkyl, Alkenyl oder Alkinyl steht,

Z für Sauerstoff, Schwefel oder einen N-Alkyl-Rest steht und

m für eine Zahl 1, 2, 3, 4, 5 oder 6 steht,

und

Ar für einen Rest

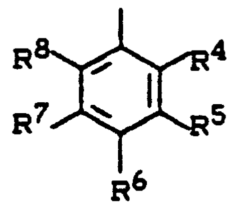

oder für einen Rest

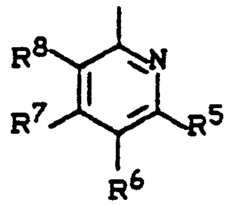

steht,
wobei
$R^4$ für Cyano, Nitro, Halogen, Alkyl, Alkoxy, Alkoxycarbonyl, Halogenalkyl, Halogenalkoxy oder für einen Rest $-S(O)_n-R^9$ steht,
$R^5$, $R^6$, $R^7$ und $R^8$ unabhängig voneinander für Wasserstoff, Cyano, Nitro, Halogen, Alkyl, Alkoxy, Alkoxycarbonyl, Halogenalkyl, Halogenalkoxy oder für einen Rest $-S(O)_n-R^9$ stehen, wobei
$R^9$ für Alkyl, Halogenalkyl, Amino, Alkylamino oder Dialkylamino steht und
n für eine Zahl 0, 1 oder 2 steht,
gefunden.

Weiterhin wurde gefunden, daß man die neuen 1-Aryl-pyrazole der allgemeinen Formel (I),

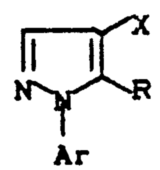

(I)

in welcher
X für Cyano oder Nitro steht,
R für Cyano oder für einen Rest -CO-Y-$R^1$ steht,
wobei
Y für Sauerstoff, Schwefel oder einen Rest $-\underset{R^2}{\underset{|}{N}}-$ steht,
$R^1$ für Wasserstoff, für Alkyl, Hydroxyalkyl, Alkoxyalkyl, Alkylthioalkyl, Halogenalkyl, Alkenyl, Halogenalkenyl, Alkinyl oder für jeweils gegebenenfalls substituiertes Cycloalkyl, Aralkyl oder Aryl steht, für einen Rest

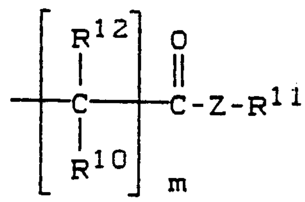

steht,

oder $R^1$ für den Fall, daß Y für Sauerstoff, Schwefel oder einen Rest $-N-SO_2-R^3$ steht, auch für ein salzartig gebundenes anorganisches oder organisches Kation steht,
$R^2$ für Wasserstoff, für Alkyl, Hydroxyalkyl, Alkoxyalkyl, Alkylthioalkyl, Halogenalkyl, Alkenyl, Alkinyl oder Halogenalkenyl, für jeweils gege benenfalls substituiertes Cycloalkyl, Aralkyl oder Aryl steht oder für einen Rest $-SO_2-R^3$ steht, wobei
$R^3$ für jeweils gegebenenfalls substituiertes Alkyl, Aralkyl oder Aryl steht,
$R^{10}$ und $R^{12}$ unabhängig voneinander für Wasserstoff oder Alkyl stehen,
$R^{11}$ für Wasserstoff, Alkyl, Alkenyl oder Alkinyl steht,
Z für Sauerstoff, Schwefel oder einen N-Alkyl-Rest steht und
m für eine Zahl 1, 2, 3, 4, 5 oder 6 steht,
und
Ar für einen Rest

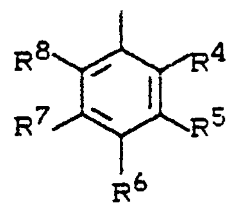

oder für einen Rest

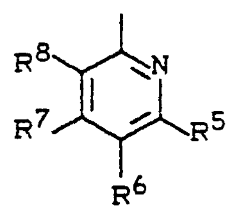

steht,
wobei
$R^4$ für Cyano, Nitro, Halogen, Alkyl, Alkoxy, Alkoxycarbonyl, Halogenalkyl, Halogenalkoxy oder für einen Rest $-S(O)_n-R^9$ steht,
$R^5$, $R^6$, $R^7$ und $R^8$ unabhängig voneinander für Wasserstoff, Cyano, Nitro, Halogen, Alkyl, Alkoxy, Alkoxycarbonyl, Halogenalkyl, Halogenalkoxy oder für einen Rest $-S(O)_n-R^9$ stehen, wobei
$R^9$ für Alkyl, Halogenalkyl, Amino, Alkylamino oder Dialkylamino steht und
n für eine Zahl 0, 1 oder 2 steht,
nach den im folgenden beschriebenen Herstellungsverfahren erhält:

(a) Man erhält 5-Carboxy-1-aryl-pyrazole der allgemeinen Formel (Ia),

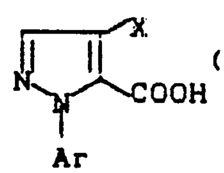 (Ia)

in welcher
X und Ar die oben angegebenen Bedeutungen haben,
wenn man
(a-α) 5-Halogen-1-aryl-pyrazole der Formel (II)

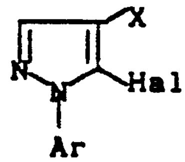 (II)

in welcher
X und Ar die oben angegebenen Bedeutungen haben und
Hal für Halogen steht,
mit Kohlendioxid in Gegenwart einer Lithium-organischen Verbindung und in Gegenwart eines Verdünnungsmittels umsetzt;
man erhält alternativ 5-Carboxy-4-nitro-1-aryl-pyrazole der Formel (Ia-1)

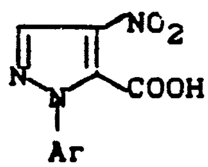 (Ia-1)

in welcher
Ar die oben angegebene Bedeutung hat,
wenn man
(a-β) 5-Methyl-4-nitro-1-aryl-pyrazole der Formel (III)

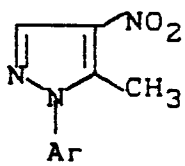 (III)

in welcher
Ar die oben angegebene Bedeutung hat,
mit Kaliumpermanganat in Gegenwart von Luftsauerstoff und in Gegenwart eines Verdünnungsmittels in bekannter Art und Weise oxidiert, oder auch wenn man
(a-γ) 5-Hydroxy-4-nitro-1-aryl-pyridazin-6-one der Formel (IV),

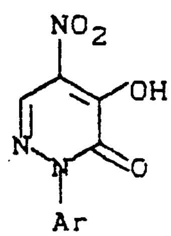 (IV)

in welcher
Ar die oben angegebene Bedeutung hat,
mit Basen in Gegenwart eines Verdünnungsmittels umsetzt; oder
(b) man erhält 5-Carboxy-1-aryl-pyrazol-Derivate der Formel (Ib-1),

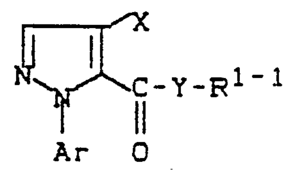

(Ib-1)

in welcher
X, Y und Ar die oben angegebene Bedeutung haben und
$R^{1-1}$ für Alkyl, Hydroxyalkyl, Alkoxyalkyl, Alkylthioalkyl, Halogenalkyl, Alkenyl, Halogenalkenyl, Alkinyl oder für jeweils gegebenenfalls substituiertes Cycloalkyl, Aralkyl oder Aryl steht, für einen Rest

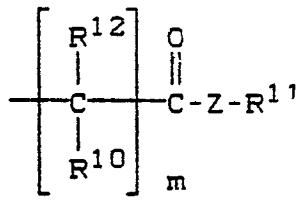

steht,
und $R^{1-1}$ für den Fall, daß Y für Schwefel oder einen Rest $-\underset{|}{N}-R^2$ steht, auch für Wasserstoff steht,

wobei
$R^{10}$ und $R^{12}$ unabhängig voneinander für Wasserstoff oder Alkyl stehen,
$R^{11}$ für Wasserstoff, Alkyl, Alkenyl oder Alkinyl steht,
Z für Sauerstoff, Schwefel oder einen N-Alkyl-Rest steht und
m für eine Zahl 1, 2, 3, 4, 5, oder 6 steht,
wenn man
(b-α) die nach Verfahren (a-α), (a-β) oder (aγ) erhältlichen 5-Carboxy-1-aryl-pyrazole der Formel (Ia)

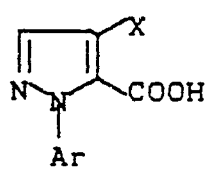

(Ia)

in welcher
X und Ar die oben angegebene Bedeutung haben, mit Alkoholen, Aminen oder Thiolen der Formel (V)
$R^{1-1}$-Y-H (V)
in welcher
$R^{1-1}$ und Y die oben angegebene Bedeutung haben,
gegebenenfalls in Gegenwart eines Reaktionshilfsmittels und gegebenenfalls in Gegenwart eines Verdünnungsmittels sowie gegebenenfalls in Gegenwart eines Säurebindemittels umsetzt;
oder man erhält Salze der Formel (Ib-2)

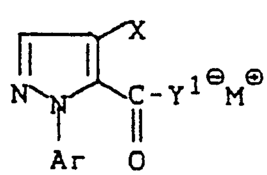

(Ib-2)

in welcher
X und Ar die oben angegebene Bedeutung haben,

Y' für Sauerstoff, Schwefel oder einen Rest $-N-SO_2-R^3$ steht und
$M^{\oplus}$ für ein anorganisches oder organisches Kation steht,
wenn man
(b-$\beta$) die nach Verfahren (a-$\alpha$), (a-$\beta$), (a-$\gamma$) und (b-$\alpha$) erhältlichen 5-Carboxy-1-aryl-pyrazol-Derivate der Formel

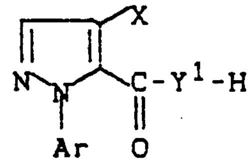

(Id)

in welcher
X, Y' und Ar die oben angegebene Bedeutung haben,
mit anorganischen oder organischen Basen gegebenenfalls in Gegenwart eines Verdünnungsmittels in üblicher Art und Weise umsetzt;
oder man erhält alternativ 1-Aryl-pyrazole der Formel (Ib-3),

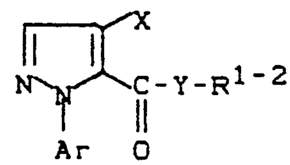

(Ib-3)

in welcher
X und Ar die oben angegebene Bedeutung haben und
$R^{1-2}$ für Alkyl, Hydroxyalkyl, Alkoxyalkyl, Alkylthioalkyl, Halogenalkyl, Alkenyl, Halogenalkenyl, Alkinyl oder für jeweils gegebenenfalls substituiertes Cycloalkyl, Aralkyl oder Aryl steht oder für einen Rest

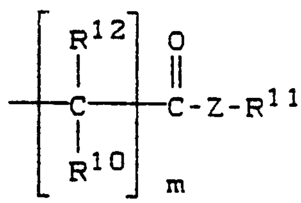

steht,
wobei
$R^{10}$ und $R^{12}$ unabhängig voneinander für Wasserstoff oder Alkyl stehen,
$R^{11}$ für Wasserstoff, Alkyl, Alkenyl oder Alkinyl steht,
Z für Sauerstoff, Schwefel oder einen N-Alkyl-Rest steht und
m für eine Zahl 1, 2, 3, 4, 5 oder 6 steht,
wenn man
(b-$\gamma$) die nach Verfahren (b-$\beta$) erhältlichen Salze der Formel (Ib-2)

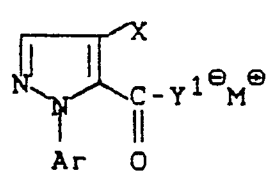

(Ib-2)

in welcher
X, Y', $M^{\oplus}$ und Ar die oben angegebene Bedeutung haben,
mit Alkylierungsmitteln der Formel (VI)
$R^{1-2}$-E (VI)
in welcher
$R^{1-2}$ die oben angegebene Bedeutung hat und
E für eine elektronenziehende Abgangsgruppe steht,
gegebenenfalls in Gegenwart eines Verdünnungsmittels umsetzt; oder

(c) man erhält 5-Cyano-1-aryl-pyrazole der Formel (Ic),

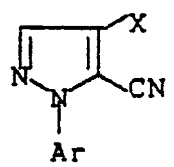

(Ic)

in welcher
X und Ar die oben angegebene Bedeutung haben,
wenn man
(c-α) die nach Verfahren (b-α) erhältlichen 5-Carbonamid-1-aryl-pyrazole der Formel (Ib-4),

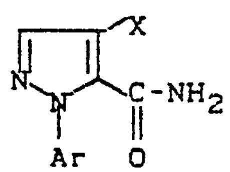

(Ib-4)

in welcher
X und Ar die oben angegebene Bedeutung haben,
mit Hilfe üblicher, allgemein bekannter Verfahren dehydratisiert,
oder wenn man
(c-β)5-Halogen-1-aryl-pyrazole der Formel (II)

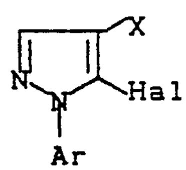

(II)

in welcher
X und Ar die oben angegebene Bedeutung haben und Hal für Halogen steht,
mit Cyaniden der Formel (VII)
$G^{\oplus}$-$CN^{\ominus}$ (VII)
in welcher
$G^{\oplus}$ für ein geeignetes Gegenion steht,
gegebenenfalls in Gegenwart eines Verdünnungsmittels und gegebenenfalls in Gegenwart eines Phasentransferkatalysators umsetzt.

Schließlich wurde gefunden, daß die neuen 1-Aryl-pyrazole der allgemeinen Formel (I), herbizide, insbesondere auch selektiv-herbizide Eigenschaften besitzen.

Überraschenderweise zeigen die erfindungsgemäßen 1-Aryl-pyrazole der Formel (I) eine erheblich bessere allgemein herbizide Wirksamkeit gegen schwer bekämpfbare Unkräuter und gleichzeitig eine deutlich verbesserte Kulturpflanzenverträglichkeit im Vergleich zu den aus dem Stand der Technik bekannten 1-Aryl-pyrazolen wie beispielsweise dem 4-Cyano-5-propionamido-1-(2,4,6-trichlorphenyl)-pyrazol, welches chemisch und wirkungsmäßig naheliegende Verbindungen sind.

Die erfindungsgemäßen 1-Aryl-pyrazole sind durch die Formel (I) allgemein definiert. Bevorzugt sind Verbindungen der Formel (I), bei welchen

X für Cyano oder Nitro steht,

R für Cyano oder für einen Rest -CO-Y-$R^1$ steht,

wobei

Y für Sauerstoff, Schwefel oder für einen Rest 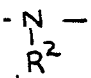 steht,

$R^1$ für Wasserstoff, für jeweils geradkettiges oder verzweigtes Alkyl, Hydroxyalkyl, Alkoxyalkyl, Alkylthioalkyl, Halogenalkyl, Alkenyl, Halogenalkenyl oder Alkinyl mit jeweils bis zu 12 Kohlenstoffatomen und im Falle des Halogenalkyl bzw. des Halogenalkenyl mit 1 bis 15 gleichen oder verschiedenen Halogenatomen, für Cycloalkyl mit 3 bis 7 Kohlenstoffatomen oder für jeweils gegebenenfalls einfach oder mehrfach, gleich oder verschieden durch Halogen, Cyano, Nitro sowie jeweils geradkettiges oder verzweigtes Alkyl, Alkoxy, Alkylthio oder Halogenalkyl mit jeweils 1 bis 4 Kohlenstoffatomen und im Fall des Halogenalkyls mit 1 bis 9 gleichen oder verschiedenen Halogenatomen substituiertes Phenyl, Benzyl oder Phenethyl steht, außerdem für einen Rest

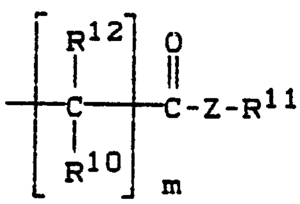

steht,

und $R^1$ für den Fall, daß Y für Sauerstoff, Schwefel oder einen Rest - N -$SO_2$-$R^3$

steht, auch für ein Äquivalent eines Alkali-, Erdalkali-, Kupfer-, Zink-, Mangan-, Zinn-,Eisen-, Cobalt-oder Nickelkations oder für ein gegebenenfalls einfach oder mehrfach, gleich oder verschieden substituiertes Ammonium-, Phosphonium-oder Sulfoniumkation steht, wobei als Substituenten in Frage kommen: geradkettiges oder verzweigtes Alkyl mit 1 bis 18 Kohlenstoffatomen, Phenyl oder Benzyl;

$R^2$ für Wasserstoff, für jeweils geradkettiges oder verzweigtes Alkyl, Hydroxyalkyl, Alkoxyalkyl. Alkylthioalkyl, Halogenalkyl, Alkenyl, Halogenalkenyl oder Alkinyl mit jeweils bis zu 12 Kohlenstoffatomen und im Falle des Halogenalkyl bzw. des Halogenalkenyl mit 1 bis 15 gleichen oder verschiedenen Halogenatomen, für Cycloalkyl mit 3 bis 7 Kohlenstoffatomen oder für jeweils gegebenenfalls einfach oder mehrfach, gleich oder verschieden durch Halogen, Cyano, Nitro sowie jeweils geradkettiges oder verzweigtes Alkyl, Alkoxy, Alkylthio oder Halogenalkyl mit jeweils 1 bis 4 Kohlenstoffatomen und im Fall des Halogenalkyls mit 1 bis 9 gleichen oder verschiedenen Halogenatomen substituiertes Phenyl, Benzyl oder Phenethyl steht, außerdem für einen Rest -$SO_2$-$R^3$ steht,

wobei

$R^3$ für jeweils geradkettiges oder verzweigtes Alkyl oder Halogenalkyl mit jeweils 1 bis 6 Kohlenstoffatomen und im Fall des Halogenalkyls mit 1 bis 9 gleichen oder verschiedenen Halogenatomen oder für gegebenenfalls einfach oder mehrfach, gleich oder verschieden substituiertes Phenyl, Benzyl oder Phenylethyl steht, wobei als Substituenten jeweils in Frage kommen: Halogen, Cyano, Nitro, jeweils geradkettiges oder verzweigtes Alkyl, Alkoxy, Alkylthio oder Halogenalkyl mit jeweils 1 bis 4 Kohlenstoffatomen und im Fall des Halogenalkyl mit 1 bis 9 gleichen oder verschiedenen Halogenatomen;

$R^{10}$ und $R^{12}$ unabhängig voneinander jeweils für Wasserstoff oder für geradkettiges oder verzweigtes Alkyl mit 1 bis 4 Kohlenstoffatomen stehen,

$R^{11}$ für Wasserstoff oder für jeweils geradkettiges oder verzweigtes Alkyl, Alkenyl oder Alkinyl mit jeweils bis zu 6 Kohlenstoffatomen steht,

Z für Sauerstoff, Schwefel oder einen N-$C_1$-$C_4$-Alkyl-Rest steht und

m für eine Zahl 1, 2, 3, 4, 5 oder 6 steht;

Ar für einen Rest

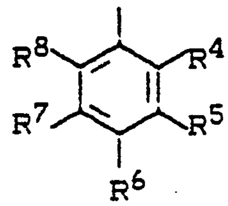

oder für einen Rest

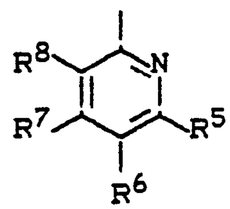

steht,
wobei
$R^4$ für Cyano, Nitro, Halogen für jeweils geradkettiges oder verzweigtes Alkyl, Alkoxy, Alkoxycarbonyl mit jeweils 1 bis 4 Kohlenstoffatomen, für jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen oder für einen Rest $-S(O)_n-R^9$ steht,
$R^5$, $R^6$, $R^7$ und $R^8$ unabhängig voneinander für Wasserstoff, Cyano, Nitro, Halogen, für jeweils geradkettiges oder verzweigtes Alkyl, Alkoxy oder Alkoxycarbonyl mit jeweils 1 bis 4 Kohlenstoffatomen, für jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen oder für einen Rest $-S(O)_n-R^9$ stehen, wobei
$R^9$ für Amino, für jeweils geradkettiges oder verzweigtes Alkyl, Alkylamino, Dialkylamino oder Halogenalkyl mit jeweils 1 bis 4 Kohlenstoffatomen in den einzelnen Alkylteilen und im Fall des Halogenalkyl mit 1 bis 9 gleichen oder verschiedenen Halogenatomen steht und
n für eine Zahl 0, 1 oder 2 steht.

Besonders bevorzugt sind Verbindungen der Formel (I), bei welchen
X für Cyano oder Nitro steht,
R für Cyano oder für einen Rest $-CO-Y-R^1$ steht, wobei Y für Sauerstoff, Schwefel oder einen Rest $-\underset{R^2}{\underset{|}{N}}-$ steht,

$R^1$ für Wasserstoff, für gebenenfalls ein-bis dreifach, gleich oder verschieden durch Fluor, Chlor, Hydroxy, Methoxy, Ethoxy, Methylthio oder Ethylthio substituiertes Methyl, Ethyl, n-oder i-Propyl, n-, i-, s-oder t-Butyl, n-oder i-Pentyl, für gegebenenfalls ein-bis dreifach, gleich oder verschieden durch Fluor oder Chlor substituiertes Allyl, Propenyl oder Butenyl, für Propargyl, Propinyl, Butinyl oder Pentinyl, für Cyclopropyl, Cylcopentyl, Cyclohexyl, für jeweils gegebenenfalls ein-bis fünffach, gleich oder verschieden durch Fluor, Chlor, Brom, Cyano, Nitro, Methyl, Ethyl, Methoxy, Ethoxy, Methylthio oder Trifluormethyl substituiertes Benzyl oder Phenyl steht, für einen Rest

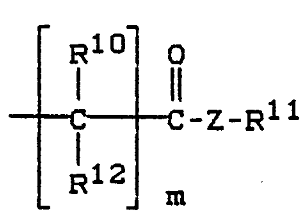

steht,

und $R^1$ für den Fall, daß Y für Sauerstoff, Schwefel oder einen Rest $-\underset{|}{N}-SO_2R^3$ steht,

auch für ein Äquivalent eines Natrium-, Kalium-, Magnesium-, Calcium-, Barium-, Kupfer-, Zink-, Mangan-, Zinn-, Eisen-, Cobalt-oder Nickelkations oder für ein gegebenenfalls ein-bis dreifach, gleich oder verschieden durch Methyl, Ethyl, n-oder i-Propyl, n-, i-, s-oder t-Butyl, Benzyl oder Phenyl substituiertes Ammonium-, Phosphonium-oder Sulfoniumkation steht,

$R^2$ für Wasserstoff, für gebenenfalls ein-bis dreifach, gleich oder verschieden durch Fluor, Chlor, Hydroxy, Methoxy, Ethoxy, Methylthio oder Ethylthio substituiertes Methyl, Ethyl, n-oder i-Propyl, n-, i-, s-oder t-Butyl, n-oder i-Pentyl, für gegebenenfalls ein-bis dreifach, gleich oder verschieden durch Fluor oder Chlor substituiertes Allyl, Propenyl oder Butenyl, für Propargyl, Propinyl, Butinyl oder Pentinyl, für Cyclopropyl, Cyclopentyl, Cyclohexyl, für jeweils gegebenenfalls ein-bis fünffach, gleich oder verschieden durch Fluor, Chlor, Brom, Cyano, Nitro, Methyl, Ethyl, Methoxy, Ethoxy, Methylthio oder Trifluormethyl substituiertes Benzyl oder Phenyl und außerdem für einen Rest

$-SO_2-R^3$ steht, wobei

$R^3$ für Methyl, Ethyl, n-oder i-Propyl, n-, i-, s-oder t-Butyl oder für gegebenenfalls ein-bis fünffach, gleich oder verschieden durch Fluor, Chlor, Brom, Cyano, Nitro, Methyl, Ethyl, Methoxy, Methylthio oder Trifluormethyl substituiertes Benzyl oder Phenyl steht,

$R^{10}$ und $R^{12}$ unabhängig voneinander jeweils für Wasserstoff, Methyl, Ethyl, n-oder i-Propyl stehen,

$R^{11}$ für Wasserstoff, Methyl, Ethyl, n-oder i-Propyl, n-, i-, s-oder t-Butyl, für Allyl, Propenyl, Butenyl, Propargyl, Propinyl oder Butinyl steht,

Z für Sauerstoff, Schwefel, einen N-Methylrest oder einen N-Ethylrest steht und

m für eine Zahl 1, 2, 3 oder 4 steht;

Ar für einen Rest

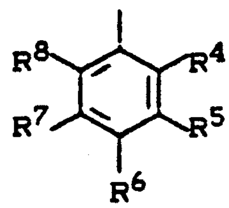

oder für einen Rest

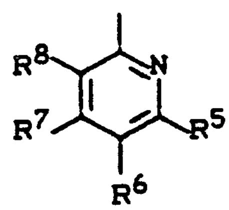

steht,

wobei

$R^4$ für Cyano, Nitro, Fluor, Chlor, Brom, Methyl, Ethyl, n-oder i-Propyl, Methoxy, Ethoxy, Trifluormethyl, Trichlormethyl, Dichlorfluormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, für Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder für einen Rest $-S(O)_n-R^9$ steht und

$R^5$, $R^6$, $R^7$ und $R^8$ unahbhängig voneinander jeweils für Wasserstoff, Cyano, Nitro, Fluor, Chlor, Brom, Methyl, Ethyl, n-oder i-Propyl, n-, i-, s-oder t-Butyl, Methoxy, Ethoxy, Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, für Trifluormethoxy, Trichlormethoxy, Dichlorfluormethoxy, Difluorchlormethoxy, Chlormethoxy, Dichlormethoxy,

Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder für einen Rest $-S(O)_n-R^9$ stehen, wobei
$R^9$ für Amino, Methylamino, Ethylamino, Dimethylamino, Diethylamino, Fluordichlormethyl, Difluormethyl, Tetrafluorethyl, Trifluorchlorethyl, Trifluormethyl, Methyl oder Ethyl steht und
n für eine Zahl 0, 1 oder 2 steht.

Ganz besonders bevorzugt ist eine Gruppe von Verbindungen der Formel (I), bei welchen
X für Cyano oder Nitro steht,
R für Cyano oder für einen Rest $-CO-Y-R^1$ steht, wobei
Y für Sauerstoff, Schwefel oder für einen Rest $-\underset{R^2}{\underset{|}{N}}-$ steht,
$R^1$ für Methyl, Ethyl, n-oder i-Propyl, n-, i-, s-oder t-Butyl, n-oder i-Pentyl, Allyl, Propenyl, n-oder i-Butenyl, n-oder i-Pentenyl, Propargyl, Propinyl, n-oder i-Butinyl, n-oder i-Pentinyl, Trifluorethyl, Trichlorethyl, Chlorethyl, Chlorpropenyl, Dichlorpropenyl, Hydroxyethyl, Hydroxypropyl, Methoxymethyl, Methoxyethyl, Methoxypropyl, Ethoxymethyl, Ethoxyethyl, Ethoxypropyl, Propoxymethyl, Propoxyethyl, Butoxymethyl, Butoxyethyl, Methylthiomethyl, Methylthioethyl, Methylthiopropyl, Ethylthioethyl, Ethylthiopropyl oder Propylthioethyl steht, und außerdem für den Fall, daß Y für einen Rest $-\underset{|}{N}-SO_2-R^3$ steht,
$R^1$ auch für ein Natrium-oder Kaliumion oder für ein gegebenenfalls ein-bis vierfach, gleich oder verschieden durch Methyl, Ethyl, n-oder i-Propyl, n-Butyl oder Benzyl substituiertes Ammoniumion steht;
$R^2$ für Wasserstoff, Methyl, Ethyl, n-oder i-Propyl, n-, i-, s-oder t-Butyl, n-oder i-Pentyl, Allyl, Propenyl, n-oder i-Butenyl, n-oder i-Pentenyl, Propargyl, Propinyl, n-oder i-Butinyl, n-oder i-Pentinyl, Trifluorethyl, Trichlorethyl, Chlorethyl, Chlorpropenyl, Dichlorpropenyl, Hydroxyethyl, Hydroxypropyl, Methoxymethyl, Methoxyethyl, Methoxypropyl, Ethoxymethyl, Ethoxyethyl, Ethoxypropyl, Methylthiomethyl, Methylthioethyl, Methylthiopropyl, Ethylthioethyl oder Ethylthiopropyl steht, oder für einen Rest $-SO_2-R^3$ steht, wobei
$R^3$ für Methyl, Ethyl, p-Tolyl oder Phenyl steht und
Ar für einen Rest

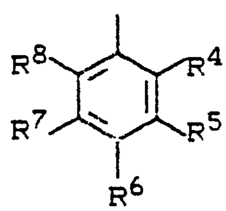

oder für einen Rest

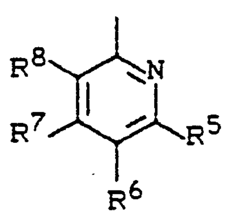

steht,
wobei
$R^4$ für Fluor, Brom oder Chlor steht,
$R^5$, $R^7$ und $R^8$ unabhängig voneinander jeweils für Wasserstoff, Fluor oder Chlor stehen und
$R^6$ für Brom, Chlor, Methyl, i-Propyl, Trifluormethyl, Fluordichlormethyl, Trifluormethoxy oder für einen Rest $-S(O)_n-R^9$ steht, wobei
$R^9$ für Methyl oder Trifluormethyl steht und
n für eine Zahl 0, 1 oder 2 steht.

Ganz besonders bevorzugt ist eine andere Gruppe von Verbindungen der Formel (I), bei welchen
X für Cyano oder Nitro steht,
R für einen Rest -CO-Y-R' steht,
wobei
Y für Sauerstoff steht und
R' für einen Rest

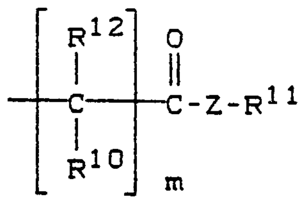

steht,
wobei
$R^{10}$ und $R^{12}$ unabhängig voneinander jeweils für Wasserstoff, Methyl oder Ethyl stehen,
$R^{11}$ für Wasserstoff, Methyl, Ethyl, n-oder i-Propyl, n-, i-, s-oder t-Butyl, für Allyl, Propenyl, Butenyl Propargyl, Propinyl oder Butinyl steht,
Z für Sauerstoff, Schwefel, eine N-Methyl-oder N-Ethylgruppe steht und
m für eine Zahl 1 oder 2 steht und
Ar für einen Rest

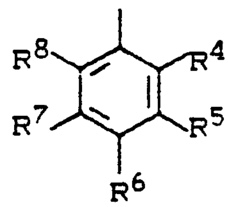

oder für einen Rest

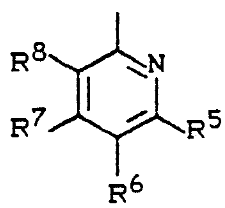

steht,
wobei
$R^4$ für Fluor, Chlor oder Brom steht,
$R^5$, $R^7$ und $R^8$ unabhängig voneinander für Wasserstoff, Fluor, Chlor oder Brom stehen und
$R^6$ für Chlor, Brom, Methyl, Ethyl, i-Propyl, Trifluormethyl, Fluordichlormethyl, Trifluormethoxy oder für einen Rest $-S(O)_n-R^9$ steht, wobei
$R^9$ für Methyl oder Trifluormethyl steht und
n für eine Zahl 0, 1 oder 2 steht.

Ganz besonders bevorzugt sind außerdem Verbindungen der Formel (I), bei welchen
X für Cyano oder Nitro steht,
R für einen Rest -CO-Y-R' steht,
wobei
Y für Sauerstoff oder Schwefel steht und

$R^1$ für Wasserstoff oder für ein Äquivalent eines Natrium-, Kalium-, Calcium-, Magnesium-, Barium-, Kupfer-, Zink-, Mangan-, Zinn-, Eisen-, Cobalt-oder Nickelkations steht oder für ein gegebenenfalls ein-bis vierfach, gleich oder verschieden durch Methyl, Ethyl, n-oder i-Propyl, n-, i-oder s-Butyl, Benzyl oder Phenyl substituiertes Ammoniumkation steht und

Ar für einen Rest

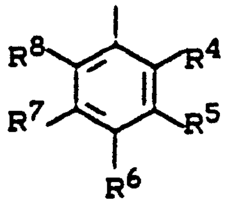

oder für einen Rest

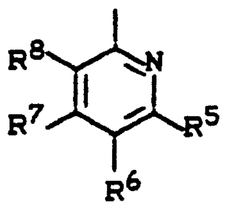

steht,

wobei

$R^4$ für Cyano, Nitro, Fluor, Chlor, Brom, Methyl, Ethyl, n-oder i-Propyl, n-, i-, s-oder t-Butyl, für Methoxy, Ethoxy, Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder für einen Rest $-S(O)_n-R^9$ steht,

$R^5$, $R^6$, $R^7$ und $R^8$ unabhängig voneinander jeweils für Wasserstoff, Cyano, Nitro, Fluor, Chlor, Brom, Methyl, Ethyl, n-oder i-Propyl, n-, i-, s-oder t-Butyl, für Methoxy, Ethoxy, Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder für einen Rest $-S(O)_n-R^9$ stehen,

wobei

$R^9$ für Amino, Methylamino, Ethylamino, Dimethylamino, Diethylamino, Fluordichlormethyl, Difluormethyl, Tetrafluormethyl, Trichlorethyl, Trifluormethyl, Methyl oder Ethyl steht

und

n für eine Zahl 0, 1 oder 2 steht.

Im einzelnen seien außer den bei den Herstellungsbeispielen genannten Verbindungen die in der folgenden Tabelle aufgeführten 1-Aryl-pyrazole der allgemeinen Formel (I) genannt:

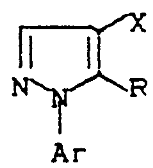 (I)

Tabelle 1:

| X | R | Ar |
| --- | --- | --- |
| CN | CN | 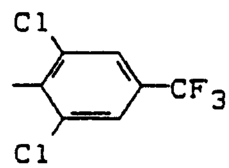 |
| CN | CN | 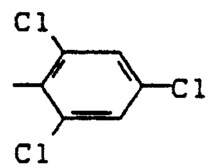 |
| $NO_2$ | CN | 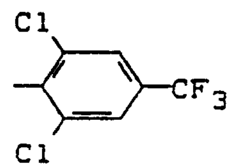 |
| $NO_2$ | CN | 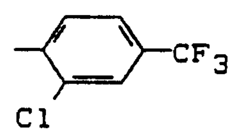 |
| $NO_2$ | CN | 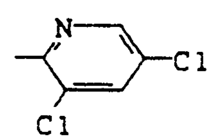 |

Tabelle 1 (Fortsetzung)

| X | R | Ar |
|---|---|---|
| CN | COOH | 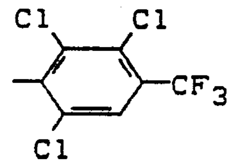 |
| CN | $-COO(CH_2)_3CH_3$ | 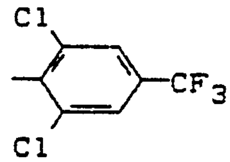 |
| $NO_2$ | $-COO(CH_2)_2-CH(CH_3)_2$ | 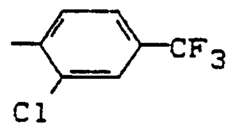 |
| CN | $-COOCH_2-CH=CH_2$ | 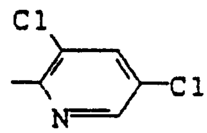 |
| $NO_2$ | $-COOCH_2-\underset{Cl}{\underset{\vert}{C}}=CH_2$ | 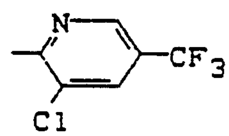 |
| CN | $-COOCH_2-CF_3$ | 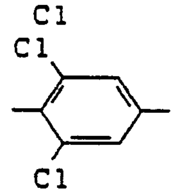 |
| $NO_2$ | $-COO-CH_2-C{\equiv}CH$ | 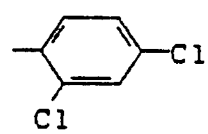 |
| CN | $-COO-CH_2-CH_2-OH$ | 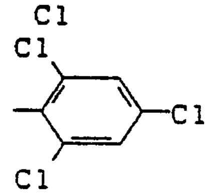 |

Tabelle 1 (Fortsetzung)

| X | R | Ar |
|---|---|---|
| $NO_2$ | $-COO-CH_2-CH_2-OCH_3$ | 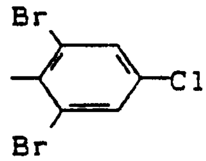 |
| CN | $-COO-CH_2-CH_2-OC_2H_5$ | 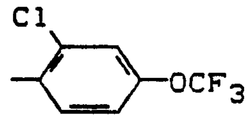 |
| $NO_2$ | $-COO-CH_2-CH_2-SCH_3$ | 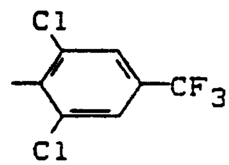 |
| CN | $-COO-CH_2-SCH_3$ | 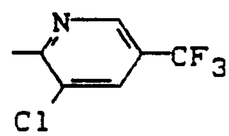 |
| $NO_2$ | $-COO-CH_2-OCH_3$ | 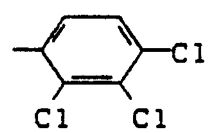 |
| $NO_2$ | -COO 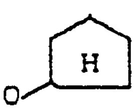 | 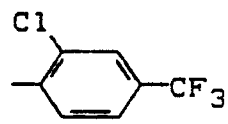 |
| CN | $-CO-NH-(CH_2)_2CH_3$ | 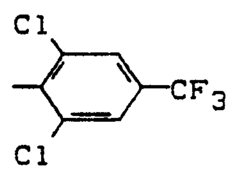 |
| $NO_2$ | $-CO-N\langle\begin{matrix}(CH_2)_2-CH_3\\(CH_2)_2-CH_3\end{matrix}$ | 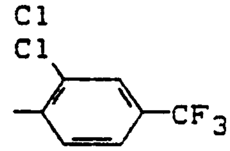 |
| CN | $-CO-NH-CH(CH_3)_2$ | 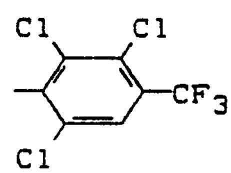 |

Tabelle 1 (Fortsetzung)

| X | R | Ar |
|---|---|---|
| $NO_2$ | $-CO-NH-SO_2CH_3$ | 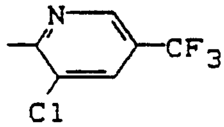 |
| CN | $-COO-CH_2-COOC_2H_5$ | 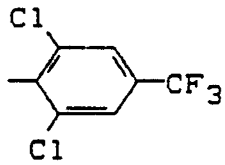 |
| $NO_2$ | $-COO-CH_2-CH_2-COOC_2H_5$ | 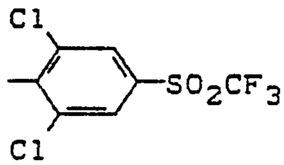 |
| CN | $-COO-CH_2-CO-NH-CH_3$ | 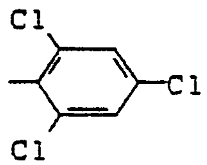 |
| $NO_2$ | $-COO^{\ominus}\ H_3N^{\oplus}-CH_2-CH(CH_3)_2$ | 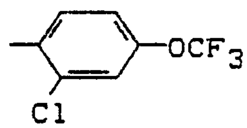 |
| $NO_2$ | $-COO^{\ominus}\ 1/2\ Hg^{2\oplus}$ | 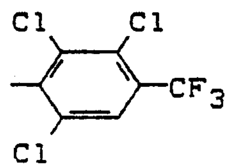 |
| $NO_2$ | $-CO-S-CH_3$ | 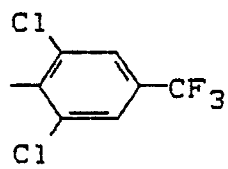 |
| CN | $-CO-S-(CH_2)_2CH_3$ | 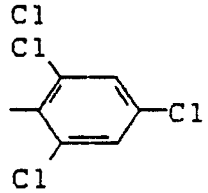 |
| $NO_2$ | $-CO-S-CH(CH_3)_2$ | 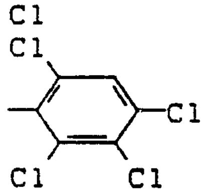 |

Verwendet man beispielsweise 5-Brom-4-nitro-1-(2,6-dichlor-4-triflourmethyl-phenyl)-pyrazol und Kohlendioxid als Ausgangsstoffe, so läßt sich der Reaktionsablauf des erfindungsgemäßen Verfahrens (a-α) durch das folgende Formelschema darstellen:

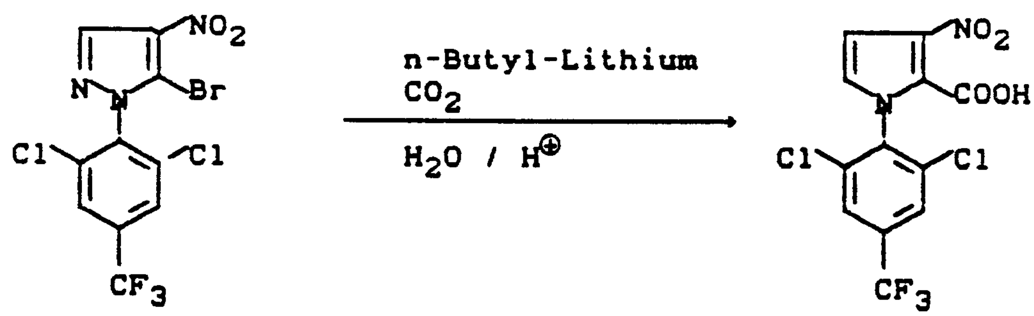


Verwendet beispielsweise 5-Methyl-4-nitro-1-(2-chlor-4-trifluormethyl-phenyl)-pyrazol als Ausgangsverbindung und Kaliumpermanganat als Oxidationsmittel, so läßt sich der Reaktionsablauf des erfindungsgemäßen Verfahrens (a-β) durch das folgende Formelschema darstellen:

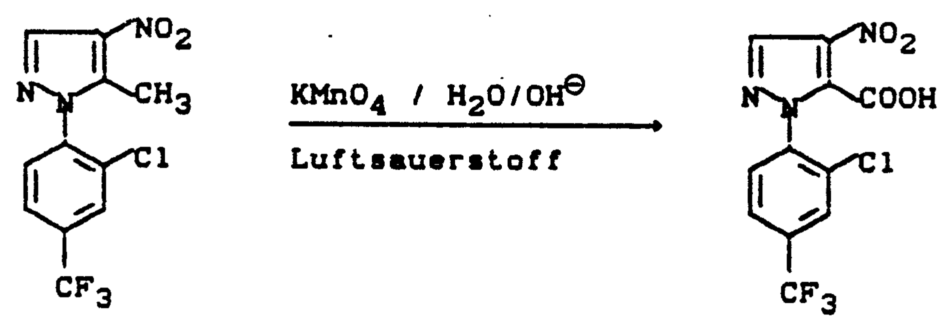


Verwendet man beispielsweise 5-Hydroxy-4-nitro-1-(2,4,6-trichlorphenyl)-pyridazin-6-on als Ausgangsverbindung und Natriumhydroxid als Katalysator, so läßt sich der Reaktionsablauf des erfindungsgemäßen Verfahrens (a-γ) durch das folgende Formelschema darstellen:

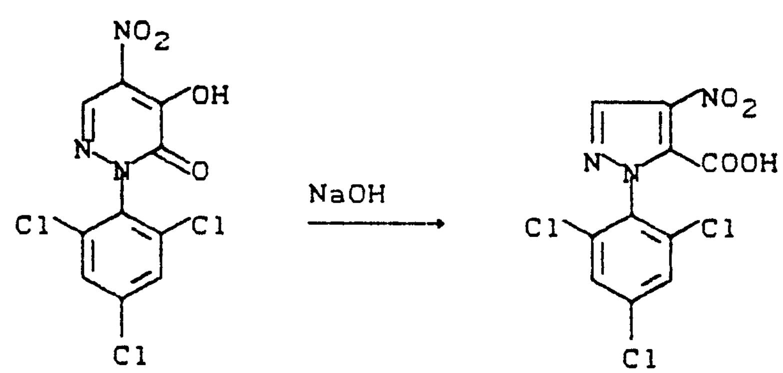


Verwendet man beispielsweise 5-Carboxy-4-cyano-1-(3,5-dichlorpyrid-2-yl)-pyrazol und Methanol als Ausgangsstoffe sowie Thionylchlorid als Reaktionshilfsmittel, so läßt sich der Reaktionsablauf des erfindungsgemäßen Verfahrens (b-α) durch das folgende Formelschema darstellen:

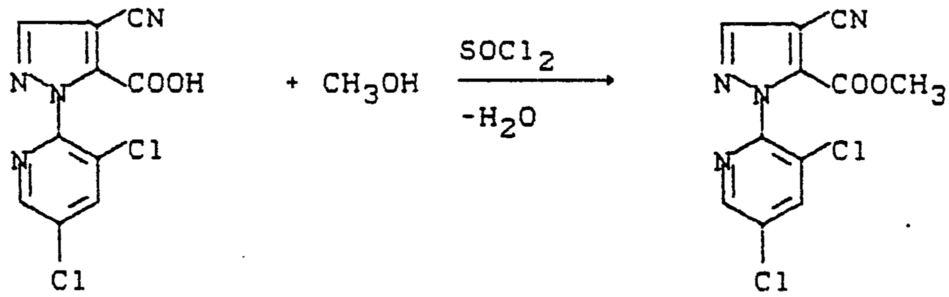


Verwendet man beispielsweise 4-Cyano-1-(2,6-dichlor-4-trifluormethyl-phenyl)-pyrazol-5-yl-thiocarbonsäure und Natriumhydroxid als Ausgangsstoffe, so läßt sich der Reaktionsablauf des erfindungsgemäßen Verfahrens (b-β) durch das folgende Formelschema darstellen:

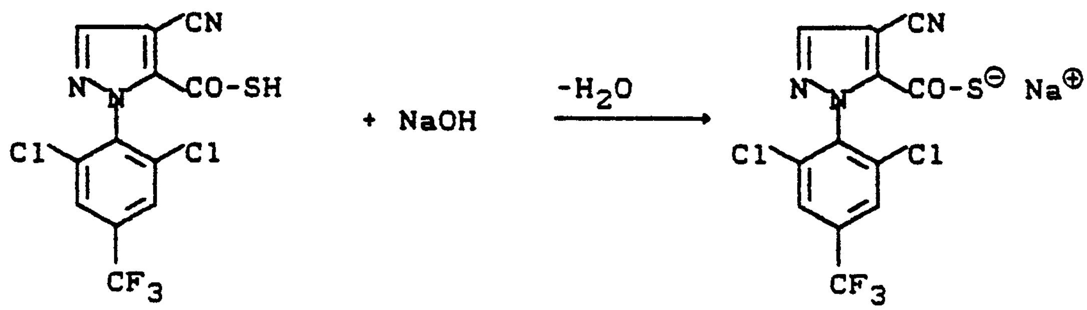


Verwendet man beispielsweise 4-Cyano-1-(2,4-dichlorphenyl)-pyrazol-5-carbonsäure Kaliumsalz und 2-Brompropionsäuremethylester als Ausgangsstoffe, so läßt sich der Reaktionsablauf des erfindungsgemäßen Verfahrens (b-γ) durch das folgende Formelschema darstellen:

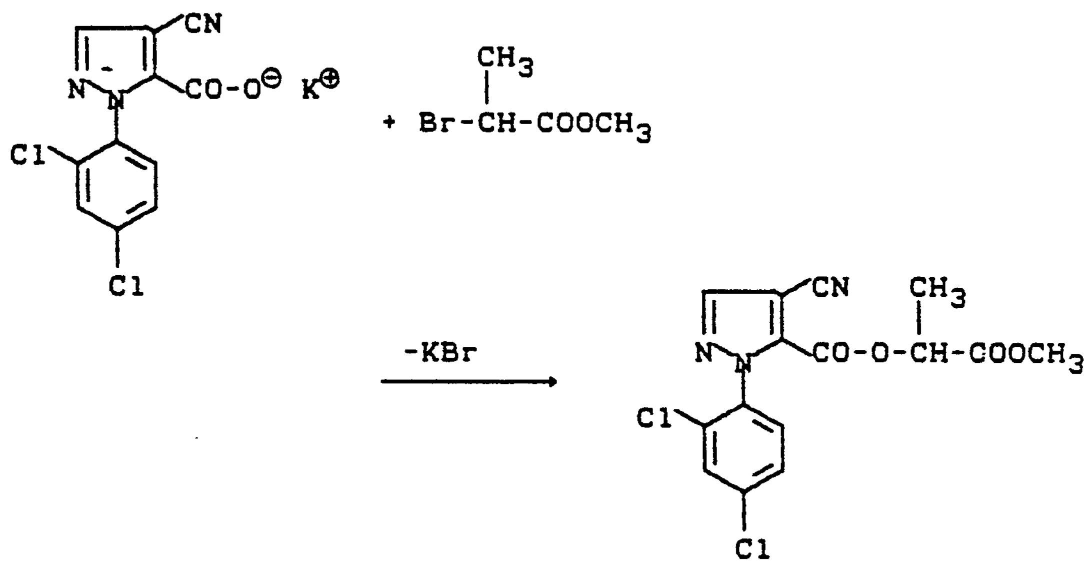


Verwendet man beispielsweise 4-Nitro-1-(2,6-dichlor-4-trifluormethylphenyl)-pyrazol-5-carbonsäureamid als Ausgangsverbindung und Phosphoroxychlorid als Dehydratisierungsmittel, so läßt sich der Reaktionsablauf des erfindungsgemäßen Verfahrens (c-α) durch das folgende Formelschema darstellen:

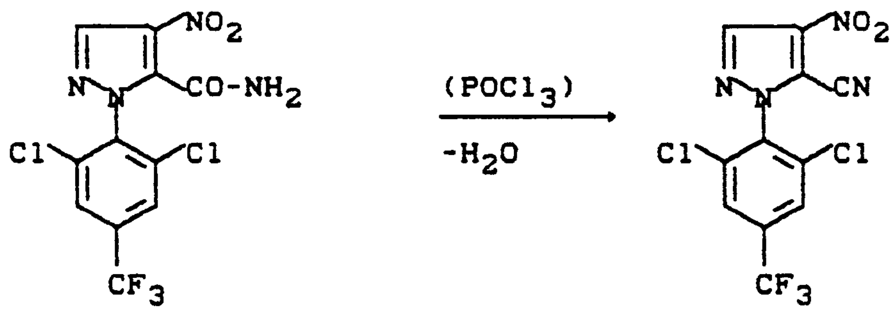


Verwendet man beispielsweise 5-Chlor-4-nitro-1-(2,4,6-trichlorphenyl)-pyrazol und Tetrabutylammoniumcyanid als Ausgangsstoffe, so läßt sich der Reaktionsablauf des erfindungsgemäßen Verfahrens (c-β) durch das folgende Formelschema darstellen:

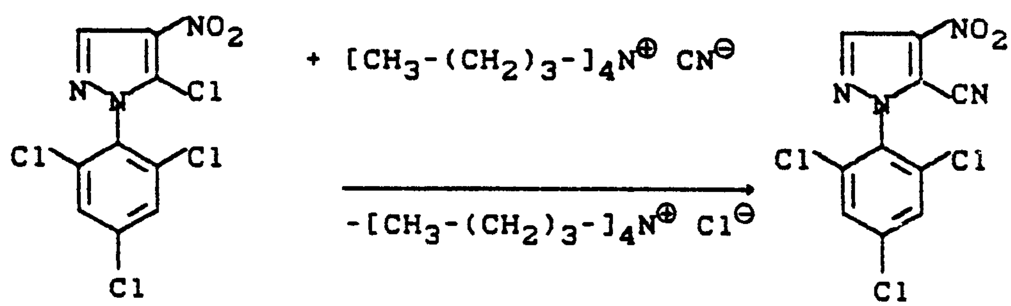


Die zur Durchführung der erfindungsgemäßen Verfahren (a-α) und (c-β) als Ausgangsstoffe benötigten 5-Halogen-1-aryl-pyrazole sind durch die Formel (II) allgemein definiert. In dieser Formel (II) stehen X und Ar vorzugsweise für diejenigen Reste, die bereits im Zusammenhang mit der Beschreibung der erfindungsgemäßen Stoffe der Formel (I) als bevorzugt für diese Substituenten genannt wurden.
Hal steht vorzugsweise für Chlor oder Brom.

Die 5-Halogen-1-aryl-pyrazole der Formel (II) sind noch nicht bekannt. Sie sind jedoch Gegenstand der eigenen vorgängigen, noch nicht veröffentlichten Patentanmeldungen DE-P 3 501 323 vom 17.1.1985 und DE-P 3 520 329 vom 7.6.1985.

Man erhält sie beispielsweise, wenn man 5-Amino-1-arylpyrazole der Formel (VIII),

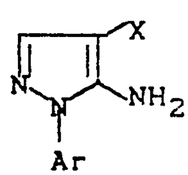 (VIII)

in welcher
X und Ar die oben angegenbene Bedeutung haben,
mit Nitritverbindungen der Formel (IX),
$R^{13}$-O-N=O (IX)
in welcher
$R^{13}$ für Wasserstoff, Alkyl oder für ein Alkalimetallkation steht,
in Gegenwart einer Halogenwasserstoffsäure wie beispielsweise Chlorwasserstoffsäure oder Bromwasserstoffsäure oder in Gegenwart eines Haloforms wie beispielsweise Chloroform oder Bromoform bei Temperaturen zwischen -20°C und +80°C in üblicher Art und Weise diazotiert (vgl. z.B. "Organikum" 15.Auflage VEB Deutscher Verlag der Wissenschaften, Berlin 1981 S. 652 ff; J. Chem. Soc. C, 1966, 1249 oder Rev. Latinoam. Quim. 13, 100-102 [1982]).

Die 5-Amino-1-aryl-pyrazole der Formel (VIII) sind teilweise bekannte (vgl. z.B. EP 26 034, EP 53 678 oder EP 34 945 sowie DE-OS 3 226 496, DE-OS 3 408 727, DE-OS 3 420 985 oder DE-OS 3 402 308), teilweise sind sie Gegenstand eigener noch nicht vorveröffentlicher Patentanmeldungen (DE-P 3 520 327 vom 7.6.85 oder DE-P 3 520 330 vom 7.6.85).

Man erhält sie beispielsweise, wenn man Aryl-hydrazine der Formel (X),

$Ar-NH-NH_2$ (X)

in welcher

Ar die oben angegebene Bedeutung hat,

($\alpha$) mit Acrylnitril-Derivaten der Formel (XIa),

$$E^1-CH=C\left\langle{}^{CN}_{X}\right. \qquad (XIa)$$

in welcher

X die oben angegebene Bedeutung hat und

$E^1$ für Halogen, Hydroxy, Alkoxy oder Dialkylamino steht,

oder

($\beta$) mit 2-Halogenacrylnitrilen der Formel (XIb),

$$CH_2=C\left\langle{}^{CN}_{Hal^1}\right. \qquad (XIb)$$

in welcher

$Hal^1$ für Halogen, insbesondere für Chlor oder Brom steht,

zunächst in einer 1. Stufe, gegebenenfalls in Gegenwart eines Verdünnungsmittels wie beispielsweise Eisessig oder Ethanol sowie gegebenenfalls in Gegenwart eines Reaktionshilfsmittels wie beispiels weise Natriumacetat bei Temperaturen zwischen -20° C und +20° C umsetzt zu den Arylhydrazin-Derivaten der Formel (XII),

$$Ar-NH-NH-CH=C\left\langle{}^{CN}_{X^1}\right. \qquad (XII)$$

in welcher

Ar die oben angegebene Bedeutung hat und

$X^1$ für Halogen, Cyano oder Nitro steht

und diese in einer 2. Stufe, gegebenenfalls in Gegenwart eines Verdünnungsmittels wie beispielsweise Ethylenglykolmonoethylether und gegebenenfalls in Gegenwart eines sauren Katalysators wie beispielsweise Schwefelsäure oder Phosphorsäure bei Temperaturen zwischen +50° C und +150° C cyclisiert.

Die Arylhydrazine der Formel (X) können auch direkt in einem Reaktionsschritt, ohne Isolierung der Zwischenstufe der Formel (XII) gegebenenfalls in Gegenwart eines Verdünnungsmittels beispielsweise Ethylenglykolmonoethylether oder Ethanol bei Temperaturen zwischen +50° C und +150° C cyclisiert werden. Gegebenenfalls werden die nach der Variante ($\beta$) erhältlichen in 4-Stellung unsubstituierten 5-Aminopyrazole der Formel (XIII),

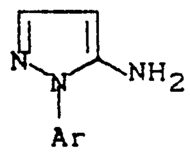

(XIII)

in welcher
Ar die oben angegebene Bedeutung hat,
in einer Folgereaktion mit einem Nitrierungsmittel wie beispielsweise Salpetersäure gegebenenfalls in Gegenwart eines Verdünnungsmittels wie beispielsweise Eisessig und gegebenenfalls in Gegenwart eines Reaktionshilfsmittels wie beispielsweise Acetanhydrid bei Temperaturen zwischen -20°C und +50° C nitriert.

Dabei kann es gegebenenfalls von Vorteil sein, vor der Nitrierungsreaktion die Aminogruppe in der 5-Position des Pyrazolringes mit Hilfe üblicher Schutzgruppentechnik, beispielsweise durch Acylierung zu - schützen und die Aminoschutzgruppe nach erfolgter Nitrierung ebenfalls in üblicher Art und Weise beispielsweise durch Verseifung mit wässriger oder alkoholischer Base wieder abzuspalten.

Die Arylhydrazine der Formel (X) sind bekannt (vgl. z.B. US-PS 4 127 575; US-PS 3 609 158; DE-OS 2 558 399; J. Chem. Soc. C, 1971, 167-174) oder können nach bekannten Verfahren in einfacher analoger Weise erhalten werden (vgl. z.B. Houben-Weyl "Methoden der organischen Chemie" Band X/2, S. 203, Thieme Verlag Stuttgart, 1967), indem man beispielsweise die bekannten Aniline bzw. Pyridylamine der Formel (XIV),

$Ar-NH_2$ (XIV)

in welcher
Ar die oben angegebene Bedeutung hat,
mit Natriumnitrit in Gegenwart einer Säure wie beispielsweise Schwefelsäure, und dann mit Zinn-II-chlorid ebenfalls in Gegenwart einer Säure wie beispielsweise Salzsäure bei Temperaturen zwischen -20° C und +80° C umsetzt oder wenn man Halogenaromaten der Formel (XV),

$Ar-Hal^2$ (XV)

in welcher
Ar die oben angegebene Bedeutung hat und
$Hal^2$ für Halogen, insbesondere für Fluor, Chlor oder Brom steht,
mit Hydrazinhydrat gegebenenfalls in Gegenwart eines Verdünnungsmittels wie beispielsweise Pyridin oder Dioxan bei Temperaturen zwischen 0° und 150° C umsetzt.

Die Nitritverbindungen der Formel (IX), die AcrylnitrilDerivate der Formel (XIa), die 2-Halogenacrylnitrile der Formel (XIb), die Aniline und Pyridylamine der Formel (XIV) und Halogenaromaten der Formel (XV) sind allgemein bekannte Verbindungen der organischen Chemie.

Die zur Durchführung des erfindungsgemäßen Verfahrens (a-β) als Ausgangsstoffe benötigten 5-Methyl-4-nitro-1-aryl-pyrazole sind durch die Formel (III) allgemein definiert. In dieser Formel (III) steht Ar vorzugsweise für diejenigen Reste, die bereits im Zusammenhang mit der Beschreibung der erfindungsgemäßen Stoffe der Formel (I) als bevorzugt für diesen Substituenten genannt wurden.

Die 5-Methyl-4-nitro-1-aryl-pyrazole der Formel (III) sind noch nicht bekannt. Sie sind jedoch teilweise Gegenstand der eigenen vorgängigen noch nicht veröffentlichten Patentanmeldung DE-P 3 509 567 vom 16.03.85.

Die 5-Methyl-4-nitro-1-aryl-pyrazole der Formel (IIIa),

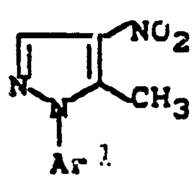

in welcher
$Ar^1$ für einen Rest

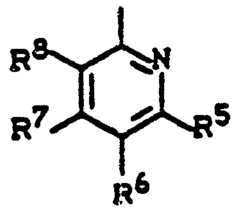

steht,
wobei
$R^5$, $R^6$, $R^7$ und $R^8$ unabhängig voneinander für Wasserstoff, Cyano, Nitro, Halogen, Alkyl, Alkoxy, Alkoxycarbonyl, Halogenalkyl, Halogenalkoxy oder für einen Rest $-S(O)_n-R^9$ stehen, wobei
$R^9$ für Alkyl, Halogenalkyl, Amino, Alkylamino oder Dialkylamino steht und
n für eine Zahl 0, 1 oder 2 steht,
sind neu.

In der Formel (IIIa) stehen $R^5$, $R^6$, $R^7$, $R^8$, $R^9$ und n in $Ar^1$ vorzugsweise bzw. besonders bevorzugt für diejenigen Reste, die bereits im Zusammenhang mit der Beschreibung der erfindungsgemäßen Stoffe der Formel (I) als bevorzugt bzw. besonders bevorzugt für diese Substituenten genannt wurden.

Man erhält 5-Methyl-4-nitro-1-aryl-pyrazole der Formeln (III) und (IIIa) beispielsweise, wenn man Acrylester-Derivate der Formel (XVI),

$$E^2\text{-CH=C}\Big\langle\begin{matrix}\text{COOR}^{14}\\ \text{CO-CH}_3\end{matrix} \qquad \text{(XVI)}$$

in welcher
$E^2$ für Alkoxy, insbesondere Methoxy oder Ethoxy oder für Dialkylamino, insbesondere für Dimethylamino steht und
$R^{14}$ für Alkyl, insbesondere für Methyl oder Ethyl steht,
mit Arylhydrazinen der Formel (X),
$Ar\text{-}NH\text{-}NH_2$ (X)
in welcher
Ar die oben angegebene Bedeutung hat,
zunächst in einer ersten Stufe, gegebenenfalls in Gegenwart eines Verdünnungsmittels, wie bespielsweise Ethanol oder Diethylether und gegebenenfalls in Gegenwart eines Reaktionshilfsmittels wie beispielsweise Chlorwasserstoffsäure bei Temperaturen zwischen -20° C und +20° C umsetzt zu den Arylhyrazin-Derivaten der Formel (XVII),

$$\text{Ar-NH-NH-CH=C}\Big\langle\begin{matrix}\text{COOR}^{14}\\ \text{CO-CH}_3\end{matrix} \qquad \text{(XVII)}$$

in welcher
Ar und $R^{14}$ die oben angegebene Bedeutung haben,
und diese in einer 2.Stufe gegebenenfalls in Gegenwart eines Verdünnungsmittels, wie beispielsweise Ethanol und gegebenenfalls in Gegenwart eines Katalysators, wie beispielsweise Schwefelsäure, bie Temperaturen zwischen +50° C und +150° C cyclisiert.

Die 5-Methyl-4-nitro-1-aryl-pyrazole der Formel (III) können auch direkt in einem Reaktionsschritt, ohne Isolierung der Zwischenstufe der Formel (XVII) gegebenenfalls in Gegenwart eines Verdünnungsmittels wie beispielsweise Ethanol oder Ethylenglykolmonoethylether bei Temperaturen zwischen +50° C und +150° C cyclisiert werden.

Die so erhältlichen 5-Methyl-pyrazol-4-carbonsäureester der Formel (XVIII),

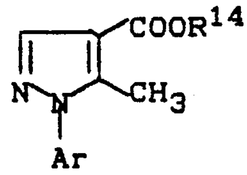

(XVIII)

in welcher

Ar und $R^{14}$ die oben angegebene Bedeutung haben,

werden in üblicher Art und Weise beispielsweise mit Basen wie Natriumhydroxid oder mit Säuren wie Bromwasserstoffsäure gegebenenfalls in Gegenwart eines Verdünnungsmittels wie beispielsweise Ethanol oder Wasser bei Temperaturen zwischen -20° C und +100° C verseift und die so erhältlichen 5-Methyl-pyrazol-4-carbonsäuren der Formel (XVIIIa),

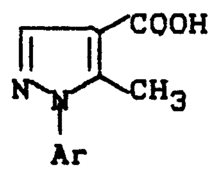

(XVIIIa)

in welcher

Ar die oben angegebene Bedeutung hat,

werden gegebenenfalls in Gegenwart eines Verdünnungsmittels wie beispielsweise Ethanol oder Wasser bei Temperaturen zwischen +100° C und +200° C decarboxyliert, wobei die Verseifung und anschließende Decarboxylierung auch in einem Reaktionsschritt als sogenanntes "Eintopfverfahren" ohne Isolierung der Zwischenprodukte der Formel (XVIIIa) durchgeführt werden können.

Schließlich werden die so erhältlichen 5-Methyl-1-aryl-pyrazole der Formel (XIX),

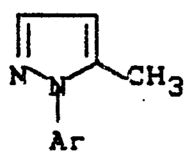

(XIX)

in welcher

Ar die oben angegebene Bedeutung hat,

mit einem Nitrierungsmittel, wie beispielsweise Salpetersäure gegebenenfalls in Gegenwart eines Verdünnungsmittels wie beispielsweise Eisessig oder Schwefelsäure und gegebenenfalls in Gegenwart eines Katalysators wie beispielsweise Acetanhydrid oder Schwefelsäure bei Temperaturen zwischen -20° C und +150° C umgesetzt.

Die Decarboxylierung der 5-Methyl-pyrazol-4-carbonsäuren der Formel (XVIIIa) und die anschließende Nitrierung der 5-Methyl-1-aryl-pyrazole der Formel (XIX) können dabei ebenfalls in einem Reaktionsschritt als sogenanntes "Eintopfverfahren" ohne Isolierung der Zwischenprodukte der Formel (XIX) durchgeführt. werden.

Die Acrylester-Derivate der Formel (XVI) sind allgemein bekannte Verbindungen der organischen Chemie (vgl. z.B. Houben-Weyl "Methoden der organischen Chemie" Band VIII, S. 623, Thieme Verlag Stuttgart 4. Auflage 1952 oder Tetrahedron Lett. 25, 3743-3746 [1984]).

Die zur Durchfürhung des erfindungsgemäßen Verfahrens (a-γ) als Ausgangsstoffe benötigten 5-Hydroxy-4-nitro-1-aryl-pyridazin-6-one sind durch die Formel (IV) allgemein definiert. In dieser Formel (IV) steht Ar vorzugsweise für diejenigen Reste, die bereits im Zusammenhang mit der Beschreibung der erfindungsgemäßen Stoffe der Formel (I) als bevorzugt für diese Substituenten genannt wurden.

Die 5-Hydroxy-4-nitro-1-aryl-pyridazin-6-one der Formel (IV) sind noch nicht bekannt.

Man erhält sie jedoch in Analogie zu prinzipiell bekannten Verfahren (vgl. DE-AS 1 195 324 und DE-AS 1 086 238), wenn man Mucohalogensäuren der Formel (XXa),

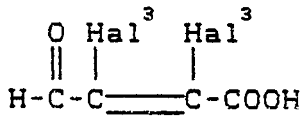

(XXa)

in welcher
Hal³ für Halogen, insbesondere für Chlor oder Brom steht, welche im Gleichgewicht mit der entsprechenden Lacton-Halbacetal-Struktur der Formel (XXb),

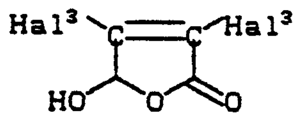

(XXb)

in welcher
Hal³ die oben angegebene Bedeutung hat,
vorliegen, mit Arylhydrazinen der Formel (X)
$Ar-NH-NH_2$ (X)
in welcher
Ar die oben angegebene Bedeutung hat,
entweder zunächst in einer ersten Stufe gegebenenfalls in Gegenwart eines Verdünnungsmittels wie beispielsweise Toluol bei Temperaturen zwischen -20° C und +20° C umsetzt zu den Hydrazonen der Formel (XXI),

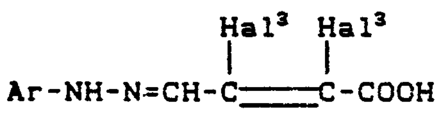

(XXI)

in welcher
Ar und Hal³ die oben angegebene Bedeutung haben,
und diese in einer zweiten Stufe gegebenenfalls in Gegenwart eines Verdünnungsmittels, wie beispielsweise Eisessig, bei Temperaturen zwischen +100° C und +150° C cyclisiert,
oder direkt in einem Reaktionsschritt, ohne Isolierung der Zwischenstufe der Formel (XXI), gegebenenfalls in Gegenwart eines Verdünnungsmittels wie beispielsweise Toluol oder Eisessig und gegebenenfalls in Gegenwart eines Katalysators wie beispielsweise p-Toluolsulfonsäure bei Temperaturen zwischen +100° C und +200° C cyclisiert und die so erhältlichen 4,5-Dihalogen-pyridazin-6-one der Formel (XXII),

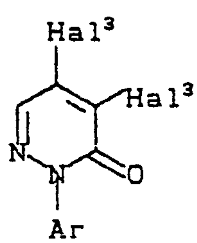

(XXII)

in welcher
Hal³ und Ar die oben angegebene Bedeutung haben,
mit Natriumnitrit gegebenenfalls in Gegenwart eines Verdünnungsmittels, wie beispielsweise Dimethylforma-

mid bei Temperaturen zwischen +50° C und 180° C umsetzt.

Die Mucohalogensäuren der Formel (XXa) bzw. (XXb) sind allgemein bekannte Verbindungen der organischen Chemie.

Die zur Durchführung des erfindungsgemäßen Verfahrens (b-α) als Ausgangsstoffe benötigten 5-Carboxy-1-aryl-pyrazole sind durch die Formel (Ia) allgemein definiert. In dieser Formel (Ia) stehen X und Ar vorzugsweise für diejenigen Reste, die bereits im Zusammenhang mit der Beschreibung der erfindungsgemäßen Stoffe der Formel (I) als bevorzugt für diese Substituenten genannt wurden.

Die 5-Carboxy-1-aryl-pyrazole der Formel (Ia) sind erfindungsgemäße Verbindungen und erhältlich mit Hilfe der erfindungsgemäßen Verfahren (a-α), (a-β) oder (a-γ).

Die zur Durchführung des erfindungsgemäßen Verfahrens (b-α) weiterhin als Ausgangsstoffe benötigten Alkohole, Amine oder Thiole sind durch die Formel (V) allgemein definiert. In dieser Formel (V) steht Y vorzugsweise für diejenigen Reste, die bereits im Zusammenhang mit der Beschreibung der erfindungsgemäßen Stoffe der Formel (I) als bevorzugt für diesen Substituenten genannt wurden.

$R^{1-1}$ steht vorzugsweise für jeweils geradkettiges oder verzweigtes Alkyl, Hydroxyalkyl, Alkoxyalkyl, Alkylthioalkyl, Halogenalkyl, Alkenyl, Halogenalkenyl oder Alkinyl mit jeweils bis zu 12 Kohlenstoffatomen und im Falle des Halogenalkyl bzw. Halogenalkenyl mit 1 bis 15 gleichen oder verschiedenen Halogenatomen, für Cycloalkyl mit 3 bis 7 Kohlenstoffatomen oder für jeweils gegebenenfalls ein fach oder mehrfach, gleich oder verschieden durch Halogen, Cyano, Nitro, jeweils geradkettiges oder verzweigtes Alkyl, Alkoxy, Alkylthio oder Halogenalkyl mit jeweils 1 bis 4 Kohlenstoffatomen und im Fall des Halogenalkyls mit 1 bis 9 gleichen oder verschiedenen Halogenatomen substituiertes Phenyl, Benzyl oder Phenethyl; außerdem für einen Rest

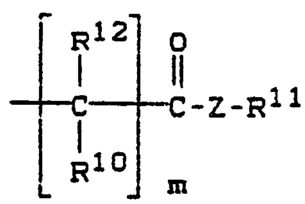

wobei

$R^{10}$ und $R^{12}$ unabhängig voneinander jeweils für Wasserstoff oder für geradkettiges oder verzweigtes Alkyl mit 1 bis 4 Kohlenstoffatomen stehen,

$R^{11}$ für Wasserstoff oder für jeweils geradkettiges oder verzweigtes Alkyl, Alkenyl oder Alkinyl mit jeweils bis zu 6 Kohlenstoffatomen steht,

Z für Sauerstoff, Schwefel oder einen N-$C_1$-$C_4$-Alkyl-Rest steht und

m für eine Zahl 1, 2, 3. 4, 5 oder 6 steht.

$R^{1-1}$ steht darüberhinaus für den Fall, daß Y für Schwefel oder für einen Rest $-\underset{|}{N}-R^2$ steht,

wobei $R^2$ vorzugs-für diejenigen Substituenten steht, die bereits im Zusammenhang mit der Beschreibung der erfindungsgemäßen Stoffe der Formel (I) als bevorzugt für diesen Rest genannt wurden, vorzugsweise auch für Wasserstoff.

Die Alkohole, Amine oder Thiole der Formel (V) sind allgemein bekannte Verbindungen der organischen Chemie.

Die zur Durchführung des erfindungsgemäßen Verfahrens (b-β) als Ausgangsstoffe benötigten 5-Carboxy-1-aryl-pyrazol-Derivate sind durch die Formel (Id) allgemein definiert. In dieser Formel (Id) stehen X und Ar vorzugsweise für diejenigen Reste, die bereits im Zusammenhang mit der Beschreibung der erfindungsgemäßen Stoffe der Formel (I) als bevorzugt für diese Substituenten genannt wurden. $Y^1$ steht vorzugsweise für Sauerstoff, Schwefel oder einen Rest $-\underset{|}{N}-SO_2-R^3$,

wobei $R^3$ vorzugsweise für diejenigen Substituenten steht, die bereits im Zusammenhang mit der Beschreibung der erfindungsgemäßen Stoffe der Formel (I) als bevorzugt für diesen Rest genannt wurden.

Die zur Durchführung des erfindungsgemäßen Verfahrens (b-γ) als Ausgangsstoffe benötigten Salze sind durch die Formel (Ib-2) allgemein definiert. In dieser Formel (Ib-2) stehen X und Ar vorzugsweise für diejenigen Reste, die bereits im Zusammenhang mit der Beschreibung der erfindungsgemäßen Stoffe der Formel (I) als bevorzugt für diese Substituenten genannt wurden, $Y^1$ steht vorzugsweise für diejenigen Reste, die bereits im Zusammenhang mit der Beschreibung der Vorprodukte der Formel (Id) als bevorzugt

für diesen Substituenten genannt wurden und $M^{\oplus}$ steht vorzugsweise für ein Äquivalent eines Natrium-, Kalium-, Magnesium-, Calcium-, Barium-, Kupfer-, Zink-, Mangan-, Zinn-, Eisen-, Cobalt-oder Nickelkations oder für ein gegebenenfalls ein-bis dreifach gleich oder verschieden durch Methyl, Ethyl, n-oder i-Propyl, n-, i-, s-oder t-Butyl, Benzyl oder Phenyl substituiertes Ammonium-, Phosphonium-oder Sulfoniumkation.

Die Salze der Formel (Ib-2) sind erfindungsgemäße Verbindungen und erhältlich mit Hilfe des erfindungsgemäßen Verfahrens (b-β).

Die zur Durchführung des erfindungsgemäßen Verfahrens (b-γ) weiterhin als Ausgangsstoffe benötigten Alkylierungsmittel sind durch die Formel (VI) allgemein definiert. In dieser Formel (VI) steht $R^{1-2}$ vorzugsweise für diejenigen Reste, die bereits im Zusammenhang mit der Beschreibung der erfindungsgemäßen Stoffe bzw. Vorprodukte der Formel (Id) als bevorzugt für diesen Substituenten genannt wurden, E steht vorzugsweise für Halogen, oder für jeweils gegebenenfalls durch Halogen substituiertes Alkylsulfonyloxy oder Alkoxysulfonyloxy oder durch Halogen oder $C_1$-$C_4$-Alkyl substituiertes Arylsulfonyloxy, insbesondere für Chlor, Brom, Iod, Methansulfonyloxy, Methoxysulfonyloxy oder p-Toluolsulfonyloxy.

Die Alkylierungsmittel der Formel (VI) sind allgemein bekannte Verbindungen der organischen Chemie.

Die zur Durchführung des erfindungsgemäßen Verfahrens (c-α) als Ausgangsstoffe benötigten 5-Carbonamid-1-aryl-pyrazole sind durch die Formel (Ib-4) allgemein definiert In dieser Formel (Ib-4) stehen X und Ar vorzugsweise für diejenigen Reste, die bereits im Zusammenhang mit der Beschreibung der erfindungsgemäßen Stoffe der Formel (I) als bevorzugt für diese Substituenten genannt wurden.

Die 5-Carbonamid-1-aryl-pyrazole der Formel (Ib-4) sind erfindungsgemäße Verbindungen und erhältlich mit Hilfe des erfindungsgemäßen Verfahrens (b-α).

Auf die zur Durchführung des erfindungsgemäßen Verfahrens (c-β) als Ausgangsstoffe benötigten 5-Halogen-1-aryl-pyrazole der Formel (II) wurde bereits bei der Beschreibung des erfindungsgemäßen Verfahrens (a-α) eingegangen.

Die zur Durchführung des erfindungsgemäßen Verfahrens (c-β) als Ausgangsstoffe weiterhin benötigten Cyanide sind durch die Formel (VII) allgemein definiert. In dieser Formel (VII) steht $G^{\oplus}$ vorzugsweise für ein Natrium-oder Kaliumkation oder für ein gegebenenfalls ein-bis vierfach, gleich oder verschieden durch Alkyl mit 1 bis 12 Kohlenstoffatomen und/oder Benzyl substituiertes Ammoniumion.

Die Cyanide der Formel (VII) sind allgemein bekannte Verbindungen der organischen Chemie.

Als Verdünnungsmittel zur Durchfürhung des erfindungsgemäßen Verfahrens (a-α) kommen inerte organische Lösungsmittel in Frage.

Hierzu gehören insbesondere aliphatische oder aromatische Kohlenwasserstoffe, wie beispielsweise Benzin, Benzol, Toluol, Xylol, Petrolether, Hexan, Cyclohexan oder Ether wie Diethylether, Dioxan, Tetrahydrofuran oder Ethylenglykoldimethyl-oder diethylether.

Das erfindungsgemäße Verfahren (a-α) wird in Gegenwart einer geeigneten Lithium-organischen-Verbindung durchgeführt. Als solche kommen alle üblicherweise für derartige Metallierungsreaktionen verwendeten Lithium-organischen-Verbindungen wie beispielsweise Methyllithium, Butyllithium oder Phenyllithium in Frage. Vorzugsweise verwendet man n-Butyllithium.

Die Reaktionstemperaturen können bei der Durchführung des erfindungsgemäßen Verfahrens (a-α) in einem gewissen Bereich variiert werden. Im allgemeinen arbeitet man bei Temperaturen zwischen -100° C und +50° C, vorzugsweise bei Temperaturen zwischen -80° C und +20° C.

Zur Durchführung des erfindungsgemäßen Verfahrens (a-α) setzt man pro Mol an 5-Halogen-1-arylpyrazol der Formel (II) im allgemeinen 1,0 bis 1,5 Mol, vorzugsweise 1,0 bis 1,3 Mol an Lithiumorganischer Verbindung und 1,0 bis 10,0 Mol, vorzugsweise 1,0 bis 5,0 Mol an gasförmigem Kohlendioxid ein.

Dabei setzt man in allgemein üblicher Art und Weise zunächst das 5-Halogen-1-aryl-pyrazol der Formel (II) unter Luft-und Feuchtigkeitsausschluß mit der Lithiumorganischen Verbindung um und leitet anschließend gasförmiges Kohlendioxid in die Reaktionsmischung ein. Die Aufarbeitung und Isolierung der Reaktionsprodukte der Formel (Ia) erfolgt nach allgemein üblichen Verfahren.

Als Verdünnungsmittel zur Durchführung des erfindunggemäßen Verfahrens (a-β) kommen insbesondere wässrige Lösungsmittel in Frage. Vorzugsweise verwendet man wässrige Natrium-oder Kaliumhydroxidlösungen.

Die Reaktionstemperaturen können bei der Durchführung des erfindungsgemäßen Verfahrens (a-β) in einem größeren Bereich variiert werden. Im allgemeinen arbeitet man bei Temperaturen zwischen 20° C und 130° C, vorzugsweise bei Temperaturen zwischen 60° C und 120° C.

Zur Durchführung des erfindungsgemäßen Verfahrens (a-β) setzt man pro Mol an 5-Methyl-4-nitro-1-aryl-pyrazol der Formel (III) im allgemeinen 1,0 bis 10,0 Mol, vorzugsweise 1,0 bis 5,0 Mol an Kaliumpermanganat ein.

Die Reaktionsdurchführung, Aufarbeitung und Isolierung der Reaktionsprodukte der Formel (Ia-1) erfolgt in üblicher Art und Weise (vgl. "Organikum" 15.Auflage, S. 433ff, VEB Deutscher Verlag der Wissenschaften, Berlin 1981; vgl. auch Herstellungsbeispiele).

Als Verdünnungsmittel zur Durchführung des erfindungsgemäßen Verfahrens (a-γ) kommen insbesondere wässrige oder mit Wasser mischbare Lösungsmittel in Frage. Vorzugsweise verwendet man Alkohole, wie Methanol oder Ethanol, Diole, wie Ethylenglykol oder Propandiol, oder deren Gemische mit Wasser, Ether, wie Tetrahydrofuran oder Dioxan, oder deren Gemische mit Wasser oder reines Wasser als Lösungsmittel.

Das erfindungsgemäße Verfahren (a-γ) wird in Gegenwart einer Base durchgeführt. Als solche kommen anorganische oder organische Basen, wie beispielsweise Lithium-, Natrium-, Kalium-, Barium-oder Calciumhydroxid oder stärker basische Amine bzw. Ammoniumbasen, wie Trimethyl-oder Triethylamin oder Benzyltrimethylammoniumhydroxid in Frage.

Die Reaktionstemperaturen können bei der Durchführung des erfindungsgemäße Verfahrens (a-γ) in einem größeren Bereich variiert werden. Im allgemeinen arbeitet man bei Temperaturen zwischen 0° C und 130° C, vorzugsweise bei Temperaturen zwischen 20° C und 110° C.

Zur Durchführung des erfindungsgemäßen Verfahrens (a-γ) setzt man pro Mol an 5-Hydroxy-4-nitro-1-aryl-pyridazin-6-on der Formel (IV) im allgemeinen 1,0 bis 10,0 Mol, vorzugsweise 1,0 bis 5,0 Mol an Base ein.

Die Reaktionsdurchführung, Aufarbeitung und Isolierung der Reaktionsprodukte der Formel (Ia-1) erfolgt in Analogie zu bekannten Verfahren (vgl. DE-AS 1 229 095 oder Chem. Pharm. Bull. 19, 1635-1640 [1971]).

Als Verdünnungsmittel zur Durchfürhrung des erfindungsgemäßen Verfahrens (b-α) kommen inerte organische Lösungsmittel in Frage.

Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether wie Diethylether, Dioxan, Tetrahydrofuran oder Ethylenglykoldimethyl-oder diethylether, Ketone wie Aceton oder Butanon, Nitrile wie Acetonitril oder Propionitril, Amide, wie Dimethylformamid, Dimethylacetamid, N-Methylformanilid, N-Methylpyrrolidon oder Hexamethylphosphorsäuretriamid, Ester wie Essigsäureethylester, oder Sulfoxide, wie Dimethylsulfoxid.

Verwendet man als Reaktionspartner der Formel (V) Alkohole, Amine oder Thiole in flüssiger Form, so ist es auch möglich, diese in einem entsprechenden Überschuß gleichzeitig als Verdünnungsmittel einzusetzen.

Das erfindungsgemäße Verfahren (b-α) wird gegebenenfalls in Gegenwart eines geeigneten Reaktionshilfsmittels durchgeführt. Als solche kommen prinzipiell alle üblichen für Veresterung und Amidierungen verwendbaren Reaktionshilfsmittel in Frage. Beispielhaft genannt seien Säurehalogenidbildner wie Thionylchlorid, Phosphortrichlorid, Phosphorpentachlorid, Phosphoroxychlorid, oder Aktivesterkomponenten wie N-Hydroxy-Succinimid, Anhydridbildner wie Chlorameisensäure-4-nitrophenylester oder übliche Kondensationsmittels wie Dicyclohexylcarbodiimid (DCC), Triphenylphosphin im Gemisch mit Tetrachlorkohlenstoff, N,N'-Carbonyldiimidazol oder N-Ethoxycarbonyl-2-ethoxy-dihydrochinolin (EEDQ).

Das erfindungsgemäße Verfahren (b-α) kann gegebenenfalls in Gegenwart eines geeigneten Säurebindemittels durchgeführt werden.

Als solche kommen alle üblichen anorganischen oder organischen Basen in Frage. Hierzu gehören beispielsweise Alkalimetallhydroxide wie Natriumhydroxdid oder Kaliumhydroxid, Alkalimetallcarbonate wie Natriumcarbonat, Kaliumcarbonat oder Natriumhydrogencarbonat, sowie tertiäre Amine wie Triethylamin, N,N-Dimethylanilin, Pyridin, N,N-Dimethylaminopyridin, Diazabicyclooctan (DABCO), Diazabicyclononen - (DBN) oder Diazabicycloundecen(DBU).

Auch ein entsprechender Überschuß eines gleichzeitig als Reaktionspartner der Formel (V) verwendeten Amins kann gegebenenfalls als Säurebindemittel dienen.

Die Reaktionstemperaturen können bei der Durchführung des erfindungsgemäßen Verfahrens (b-α) in einem größeren Bereich variiert werden. Im allgemeinen arbeitet man bei Temperaturen zwischen -20° C und +150° C, vorzugsweise bei Temperaturen zwischen 0° C und +100° C.

Zur Durchführung des erfindungsgemäßen Verfahrens (b-α) setzt man pro Mol an 5-Carboxy-1-aryl-pyrazol der Formel (Ia) im allgemeinen 1,0 bis 20,0 Mol, vorzugsweise 1,0 bis 10,0 Mol an Alkohol, Amin oder Thiol der Formel (V), 1,0 bis 5,0 Mol, vorzugsweise 1,0 bis 2,0 Mol an Reaktionshilfsmittel und gegebenenfalls 1,0 bis 2,0 Mol an Säurebindemittel ein.

In den meisten Fällen ist es vorteilhaft zunächst aus den 5-Carboxy-1-aryl-pyrazolen der Formel (Ia) und dem Reaktionshilfsmittel nach üblichen Verfahren einen aktivierten Komplex (Säurehalogenid, Aktivester, gemischtes Säureanhydrid etc.) herzustellen, welcher gegebenenfalls isoliert werden kann und entweder in einem getrennten Reaktionsschritt oder im Eintopfverfahren mit dem Alkohol, Amin oder Thiol der Formel -

(V) umgesetzt wird. Die Zugabe des Säurebindemittels kann dabei je nach dem verwendeten Reaktionshilfsmittel entweder in der 1.Stufe zur Bildung des aktivierten Komplexes oder in der 2.Stufe zur Umsetzung desselben nützlich sein. Die Reaktionsdurchführung, Aufarbeitung und Isolierung der Reaktionsprodukte der Formel (Ib-1) erfolgt nach allgemein üblichen Verfahren.

Als Verdünnungsmittel zur Durchführung des erfindungsgemäßen Verfahrens (b-β) kommen organische oder wässrige Lösungsmittel oder organisch-wässrige Lösungsmittelgemische in Frage. Vorzugsweise verwendet man Alkohole wie Methanol, Ethanol oder Propanol oder deren Gemische mt Wasser sowie reines Wasser als Verdünnungsmittel.

Das erfindungsgemäße Verfahren (b-β) wird üblicher Weise in Gegenwart einer anorganischen oder organischen Base durchgeführt. Als solche verwendet man Hydroxide, Oxide oder Carbonate von Alkali- oder Erdalkalimetallen oder geeignet substituierte Amine, in Abhängigkeit von der Art des gewünschten Gegenions $M^{\oplus}$ in den Verbindungen der Formel (Ib-2).

Die Reaktionstemperaturen können bei der Durchführung des erfindungsgemäßen Verfahrens (b-β) in einem größeren Bereich variiert werden. Im allgemeinen arbeitet man bei Temperaturen zwischen -20° C und +120° C, vorzugsweise bei Temperaturen zwischen 0° C und 80° C.

Zur Durchführung des erfindungsgemäßen Verfahrens (b-β) setzt man pro Mol an 5-Carboxy-1-arylpyrazol-Derivat der Formel (Id) im allgemeinen 1,0 bis 20,0 Mol, vorzugsweise 1,0 bis 10,0 Mol an Base ein. Die Calcium-, Barium-, Magnesium-, Mangan-, Kupfer-, Nickel-, Zinn-, Eisen-und Cobaltsalze erhält man auch aus den Natriumsalzen durch Behandeln mit einem entsprechenden anorganischen Metallsalz, z.B. Calciumchlorid, Bariumchlorid, Kupfersulfat, Nickelchlorid oder Cobaltnitrat.

Die Aufarbeitung und Isolierung der Salze der Formel (Ib-2) erfolgt nach üblichen Methoden.

Als Verdünnungsmittel zur Durchführung des erfindungsgemäßen Verfahrens (b-γ) kommen inerte organische Lösungsmittel in Frage.

Hierzu gehören insbesondere aliphatische oder aromatische, gegebenenfalls halogenierte Kohlenwasserstoffe wie beispielsweise Benzin, Benzol, Toluol, Xylol, Chlorbenzol, Petrolether, Hexan, Cyclohexan, Dichlormethan, Chloroform, Tetrachlorkohlenstoff, Ether wie Diethylether, Dioxan, Tetrahydrofuran oder Ethylenglykoldimethyl-oder diethylether, Ketone wie Aceton oder Butanon, Nitrile wie Acetonitril oder Propionitril, Amide wie Dimethylformamid, Dimethylacetamid, N-Methylformanilid, N-Methylpyrrolidon oder Hexamethylphosphorsäuretriamid, Ester wie Essigsäureethylester oder Sulfoxide wie Dimethylsulfoxid.

Die Reaktionstemperaturen können bei der Durchführung des erfindungsgemäßen Verfahrens (b-γ) in einem größeren Bereich variiert werden. Im allgemeinen arbeitet man bei Temperaturen zwischen 0° C und +180°C, vorzugsweise bei Temperaturen zwischen +20°C und 150° C.

Zur Durchführung des erfindungsgemäßen Verfahrens (b-γ) setzt man pro Mol an Salz der Formel (Ib-2) im allgemeinen 1,0 bis 10,0 Mol, vorzugsweise 1,0 bis 5,0 Mol an Alkylierungsmittel der Formel (VI) ein. Die Reaktionsdurchführung, Aufarbeitung und Isolierung der Reaktionsprodukte der Formel (Ib-3) erfolgt nach üblichen und bekannten Verfahren.

Als Dehydratisierungsmittel zur Durchführung des erfindungsgemäßen Verfahrens (c-α) kommen alle üblichen zur Amiddehydratisierung verwendbaren Systeme in Frage.

Beispielhaft genannt seien Triphenylphosphin/Tetrachlorkohlenstoff (vgl. z.B. Chem. Ber. 105, 244-256 - [1972]; Tetrahedron Letters 1970, 4383-4384); Thionylchlorid (vgl. z.B. Organic Syntheses Coll. Vol. 4 , 436-438 [1963]), p-Toluolsulfonsäurechlorid/Pyridin (vgl. z.B. J. American Chem. Soc. 77, 1701-1702 [1955]), Phosgen/Pyridin, (J. Chem. Soc. 1954, 3730); Dicyclohexylcarbodiimid (J. Org. Chemistry 26, 3356-3360 - [1961]); Phosphorpentoxid (Organic Syntheses Coll. Vol. 4, 144 [1963]), Phosphoroxychlorid/Natriumhydrogensulfit (J. Org. Chemistry 22, 1142 [1957]), Phosphorpentachlorid - (Organic Syntheses Coll. Vol. II. 379 [1943]), Acetanhydrid (J. Amer. Chem. Soc. 71, 2650-2652 [1949]) oder Oxalylchlorid/Pyridin (Synth. Commun. 10, 479-487 [1980]).

Die dabei verwendeten Verdünnungsmittel sind in der Regel abhängig vom verwendeten Dehydratisierungsmittel. Üblicherweise verwendet man inerte organische Lösungsmittel.

Hierzu gehören insbesondere aliphatische oder aromatische, gegebenenfalls halogenierte Kohlenwasserstoffe wie beispielsweise Benzin, Benzol, Toluol, Xylol, Chlorbenzol, Petrolether, Hexan, Cyclohexan, Dichlormethan, Chloroform, Tetrachlorkohlenstoff, Ether wie Diethyl ether, Dioxan, Tetrahydrofuran oder Ethylenglykoldimethyl-oder diethylether, Ketone wie Aceton oder Butanon, Nitrile wie Acetonitril oder Propionitril, Amide wie Dimethylformamide, Dimethylacetamid, N-Methylformanilid, N-Methylpyrrolidon oder Hexamethylphosphorsäuretriamid, Ester wie Essigsäureethylester oder Sulfoxide wie Dimethylsulfoxid.

Die Reaktionstemperaturen können bei der Durchführung des erfindungsgemäßen Verfahrens (c-α) in Abhängigkeit vom verwendeten Dehydratisierungssytem in größeren Bereichen variiert werden. Im allgemeinen arbeitet man bei Temperaturen zwischen -20° C und +200° C, vorzugsweise bei Temperaturen zwischen 0° C und +180° C.

Zur Durchführung des erfindungsgemäßen Verfahrens (c-α) setzt man pro Mol an 5-Carbonamid-1-aryl-pyrazol der Formel (Ib-4) in Abhängigkeit von der verwendeten Dehydratisierungsmethode im allgemeinen 1,0 bis 30,0 Mol, vorzugsweise 1,0 bis 15,0 Mol an Dehydratisierungsmittel ein. Die Aufarbeitung und Isolierung der Reaktionsprodukte der Formel (Ic) erfolgt in Analogie zu bekannten Verfahren (vgl. auch Harrison, Harrison "Compendium of Organic Synthetic Methods" Vol. 1;S.464-465 [1971]; Patai "The Chemistry of the Cyano-Group" S. 96-103 Interscience 1970; Ferri "Reaktionen der organischen Synthese" S. 240, Thieme Verlag Stuttgart 1978).

Als Verdünnungsmittel zur Durchführung des erfindungsgemäßen Verfahrens (c-β) kommen inerte organische Lösungsmittel oder organische-wässrige Zweiphasengemische in Frage.

Hierzu gehören insbesondere aliphatische oder aromatische, gegebenenfalls halogenierte Kohlenwasserstoffe wie beispielsweise Benzin, Benzol, Toluol, Xylol, Chlorbenzol, Petrolether, Hexan, Cyclohexan, Dichlormethan, Chloroform, Tetrachlorkohlenstoff, Ether wie Diethylether, Dioxan, Tetrahydrofuran oder Ethylenglykoldimethyl-oder diethylether, Ketone wie Aceton oder Butanon, Nitrile wie Acetonitril oder Propionitril, Amide wie Dimethylformamid, Dimethylacetamid, N-Methylformanilid, N-Methylpyrrolidon oder Hexamethylphosphorsäuretriamid, Ester wie Essigsäureethylester oder Sulfoxide wie Dimethylsulfoxid oder organisch-wässrige Zweiphasengemische wie Toluol-Wasser oder Dichlormethan-Wasser.

Der Zusatz von 0,01 bis 10 Gewichtsprozent (bezogen auf eingesetztes 5-Halogen-1-aryl-pyrazol der Formel (II)) eines Phasentransferkatalysators mag sich in einigen Fällen als vorteilhaft erweisen. Als Beispielse für solche Katalysatoren seien genannt:
Tetrabutylammoniumiodid, Tetrabutylammoniumbromid, Tributyl-methylphosphoniumbromid, Trimethyl-$C_{13}$/$C_{15}$-alkylammoniumchlorid, Dibenzyl-dimethylammoniummethylsulfat, Dimethyl-$C_{12}$/$C_{14}$-alkyl-benzylammoniumchlorid, Tetrabutylammoniumhydroxid, 18-Krone-6, Triethylbenzylammoniumchlorid, Trimethylbenzylammoniumchlorid.

Die Reaktionstemperaturen können bei der Durchführung des erfindungsgemäßen Verfahrens (c-β) in einem größeren Bereich variiert werden. Im allgemeinen arbeitet man bei Temperaturen zwischen 0° C und 100° C, vorzugsweise bei Temperaturen zwischen 10° C und 80° C.

Zur Durchführung des erfindungsgemäßen Verfahrens (c-β) setzt man pro Mol an 5-Halogen-1-aryl-pyrazol der Formel (II) im allgemeinen 1,0 bis 10,0 Mol, vorzugsweise 1,0 bis 5,0 Mol an Cyanid der Formel (VII) ein.
Die Reaktionsdurchführung, Aufarbeitung und Isolierung der Reaktionsprodukte der Formel (Ic) erfolgt nach allgemein üblichen Verfahren.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen:Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie-und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht-und Hopfenanlagen und zur selektiven Unkrautbekämpfung in einjährigen Kulturen eingesetzt werden.

Dabei lassen sich die erfindungsgemäßen Wirkstoffe mit besonders gutem Erfolg zur selektiven Bekämpfung von dikotylen Unkräutern in monokotylen Kulturen wie beispielsweise Mais, Weizen oder Gerste einsetzen. In entsprechenden Aufwandmengen besitzen die erfindungsgemäßen Wirkstoffe darüberhinaus insektizide und wachstumsregulatorische Wirksamkeiten.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur und synthetische Stoffe sowie Feinstverkapselungen in polymeren Stoffen.

Diese Formulierungen werden in bekannter Weise hergestellt, z..B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder - schaumerzeugenden Mitteln.

Im Falle der Benutzung von Wasser als Streckmittel können z.B. auch organische Lösungmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohle, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

Z. B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier-und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurrennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N'-dimethyl-harnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage. Auch Mischungen mit N,N-Dimethyl-N'-(3-chlor-4-methylphenyl)-harnstoff; N,N-Dimethyl-N'-(4-isopropylphenyl)-harnstoff; 4-Amino-6-t-butyl-3-ethylthio-1,2,4-triazin-5(4H)-on; 2,4-Dichlorphenoxyessigsäure; 2,4-Dichlorphenoxypropionsäure; (2-Methyl-4-chlorphenoxy)-essigsäure; (4-Chlor-2-methylphenoxy)-propionsäure; Chloressigsäure-N-(methoxymethyl)-2,6-diethylanilid; 2-Ethyl-6-methyl-N-(1-methyl-2-methoxyethyl)-chloracetanilid; 2-[4-(3,5-Dichlorpyrid-2-yloxy)-phenoxy]-propionsäure-(2-benzyloxyethylester), -(trimethylsilylmethylester) oder -(2,2-diethoxyethylester), Methyl-5-(2,4-dichlorphenoxy)-2-nitrobenzoat; 3,5-Diiod-4-hydroxybenzonitril; 3-Isopropyl-2,1,3-benzothiadiazin-4-on-2,2-dioxid; 2-Chlor-N-{[(4-methoxy-6-methyl-1,3,5-triazin-2-yl)-amino]-carbonyl}-benzolsulfonamid; 4-Ethylamino-2-t-butylamino-6-methylthio-s-triazin; N-Methyl-2-(1,3-benzthiazol-2-yloxy)-acetamid; N,N-Di-n-propyl-thiocarbamidsäure-S-ethylester; N,N-Diisopropyl-S-(2,3,3-trichlorallyl)-thiolcarbamat; N-(1-Ethylpropyl)-3,4-dimethyl-2,6-dinitroanilin; 2-Chlor-4-ethylamino-6-isopropylamino-1,3,5-triazin; 2-Chlor-4-ethylamino-6-(3-cyanopropylamino)-1,3,5-triazin; 3,5-Dibrom-4-hydroxybenzonitril; [(4-Amino-3,5-dichlor-6-fluor-2-pyridyl)-oxy]-essigsäure bzw. -1-methylheptylester sind gegebenenfalls von Vorteil. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pfanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

Herstellungsbeispiele

Beispiel 1:

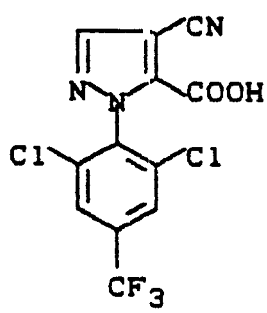

(Verfahren (a-α))

34 g (0,088 Mol) 5-Brom-4-cyano-1-(2,6-dichlor-4-trifluormethyl-phenyl)-pyrazol werden in 300 ml absolutem Ether gelöst und bei -78° C unter einer Stickstoffatmosphäre mit 60 ml (0,098 Mol) 15%igem n-Butyllithium in Hexan versetzt. Man rührt 2 Stunden bei -78° C, leitet anschließend bei -78° C einen Überschuß an gasförmigem Kohlendioxid ein und erwärmt langsam auf Raumtemperatur. Zur Aufarbeitung wird die Reaktionsmischung mit 200 ml Wasser versetzt und mit Ether zweimal gewaschen. Die wässrige Phase wird unter Eiskühlung angesäuert, dreimal mit Methylenchlorid extrahiert, die vereinigten organischen Phasen über Magnesiumsulfat getrocknet und im Vakuum eingeengt. Der Rückstand kristallisiert beim Verreiben mit Petrolether.

Man erhält 25 g (81 % der Theorie) an 5-Carboxy-4-cyano-1-(2,6-dichlor-4-trifluormethyl-phenyl)-pyrazol vom Schmelzpunkt 155-158° C.

Beispiel 2:

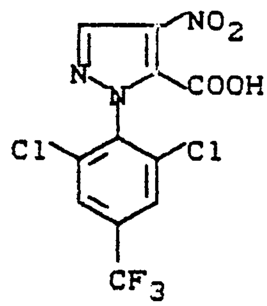

(Verfahren (a-β))

1,7 g (0,005 Mol) 5-Methyl-4-nitro-1-(2,6-dichlor-4-trifluormethyl-phenyl)-pyrazol werden in einer Lösung aus 50 ml Wasser und 4 ml 10%iger Kaliumhydroxidlösung gelöst; unter gleichzeitiger Einleitung von Luftsauerstoff setzt man portionsweise 3,6 g (0,023 Mol) Kaliumpermanganat zu und erhitzt anschließend auf Rückflußtemperatur. Die erkaltete Reaktionsmischung wird mit Methylenchlorid unterschichtet, unter Eiskühlung mit 2n-Salzsäure vorsichtig angesäuret und das ausgefallene Mangandioxid durch Natriumhydrogensulfitzugabe reduziert. Die organische Phase wird abgetrennt und mit gesättigter Natriumhydrogencarbonatlösung extrahiert. Die basischen Extrakte werden angesäuert, mehrfach mit Methylenchlorid extrahiert, die vereinigten organischen Extrakte über Magnesiumsulfat getrocknet und im Vakuum eingeengt.

Man erhält 0,34 g (18 % der Theorie) an 5-Carboxy-4-nitro-1-(2,6-dichlor-4-trifluormethyl-phenyl)-pyrazol vom Schmelzpunkt 102° C (Zersetzung).

Beispiel 3:

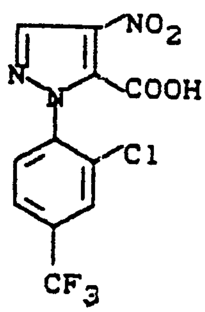

(Verfahren (a-γ))

15 g (0,0045 Mol) 5-Hydroxy-4-nitro-1-(2-chlor-4-trifluormethyl-phenyl)-pyridazin-6-on werden in 150 ml 2%iger Natronlauge 5 Minuten auf 80° C und dann 3 Minuten auf 100° C erhitzt. Man läßt auf Raumtemperatur abkühlen, filtriert den Niederschlag ab, säuert das Filtrat unter Eiskühlung vorsichtig mit 2N-Salzsäure an, extrahiert dreimal mit je 150 ml Methylenchlorid, trocknet die Methylenchloridphasen über Magnesiumsulfat und entfernt das Lösungsmittel im Vakuum.

Man erhält 11 g (73 % der Theorie) an 5-Carboxy-4-nitro-1-(2-chlor-4-trifluormethyl-phenyl)-pyrazol als ölige Substanz.

$^1$H-NMR ($CDCl_3$) $\delta = 8{,}34$ ppm (3-Pyrazol H)

Beispiel 4:

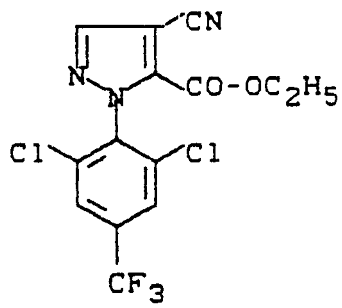

(Verfahren (b-α))

3,5 g (0,01 Mol) 5-Carboxy-4-cyano-1-(2,6-dichlor-4-trifluormethyl-phenyl)-pyrazol werden mit 19 ml Thionylchlorid 4 Stunden unter Rückfluß erhitzt. Das überschüssige Thionylchlorid wird unter vermindertem Druck abdestilliert, der Rückstand mit 15 ml absolutem Ethanol versetzt und 10 Minuten auf Rückflußtemperatur erhitzt. Die Reaktionsmischung wird im Vakuum eingeengt, der Rückstand in 50 ml Methylenchlorid aufgenommen, zweimal mit je 25 ml gesättigter Natriumhydrogencarbonatlösung gewaschen, über Magnesiumsulfat getrocknet und im Vakuum eingeengt.

Man erhält 3,2 g (85 % der Theorie) an 5-Ethoxycarbonyl--4-cyano-1-(2,6-dichlor-4-trifluormethyl-phenyl)-pyrazol vom Schmelzpunkt 99-101° C.

Beispiel 5:

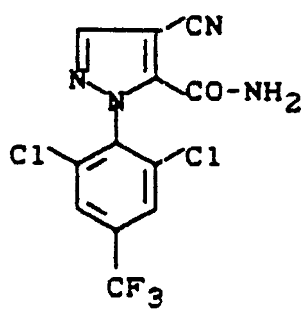

(Verfahren (b-α))

3,5 g (0,01 Mol) 5-Carboxy-4-cyano-1-(2,6-dichlor-4-trifluormethyl-phenyl)-pyrazol werden mit 10 ml Thionylchlorid 2 Stunden unter Rückfluß erhitzt. Das überschüssige Thionylchlorid wird unter vermindertem Druck abdestilliert. Der Rückstand wird bei 10° C mit 30 ml 25%igem wässrigen Ammoniak versetzt und 10 Minuten bei Raumtemperatur gerührt. Die Reaktionsmischung wird anschließend zweimal mit je 50 ml Methylenchorid extrahiert, die organische Phase mit 30 ml gesättigter Natriumhydrogencarbonatlösung gewaschen, über Magnesiumsulfat getrocknet und im Vakuum eingeengt.

Man erhält 1,3 g (37 % der Theorie) an 5-Carbamoyl-4-cyano-1-(2,6-dichlor-4-trifluormethyl-phenyl)-pyrazol vom Schmelzpunkt 166-168° C.

Beispiel 6:

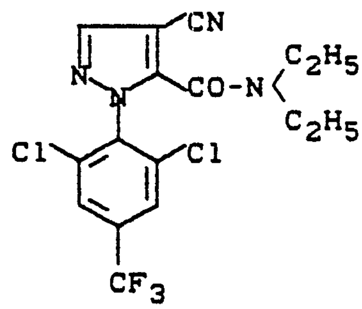

(Verfahren (b-α))

3,5 g (0,01 Mol) 5-Carboxy-4-cyano-1-(2,6-dichlor-4-trifluormethyl-phenyl)-pyrazol werden mit 15 ml Thionylchlorid 2 Stunden unter Rückfluß erhitzt. Das überschüssige Thionylchlorid wird unter vermindertem Druck abdestilliert. Der Rückstand wird in 20 ml Tetrahydrofuran gelöst, bei 10° C mit 0,7 g (0,01 Mol) Diethylamin und 0,8 g (0,01 Mol) Pyridin versetzt und 1 Stunde bei Raumtemperatur gerührt. Anschließend wird die Reaktionsmischung auf Eiswasser gegossen, dreimal mit je 80 ml Dichlormethan extrahiert, die vereinigten organischen Phasen mit gesättigter Natriumhydrogencarbonatlösung gewaschen, über Magnesiumsulfat getrocknet und im Vakuum eingeengt.

Man erhält 2,7 g (67 % der Theorie) an 5-(N,N-Diethylcarbamoyl)-4-cyano-1-(2,6-dichlor-4-trifluormethyl-phenyl)-pyrazol vom Schmelzpunkt 118-119° C.

Beispiel 7:

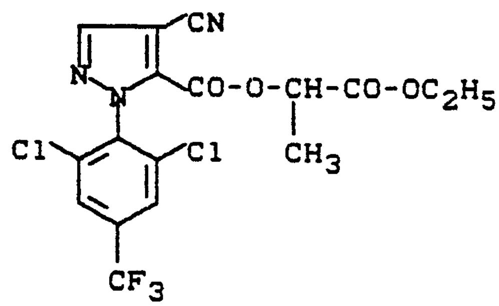

(Verfahren (b-α))

4,2 g (0,01 Mol) 5-Carboxy-4-cyano-1-(2,6-dichlor-4-trifluormethyl-phenyl)-pyrazol werden mit 20 ml Thionylchlorid 2 Stunden unter Rückfluß erhitzt. Das überschüssige Thionylchlorid wird unter vermindertem Druck abdestilliert. Den Rückstand löst man in 15 ml Dimethoxyethan, versetzt mit 1,4 g (0,012 Mol) Milchsäureethylester und 0,9 g (0,012 Mol) Pyridin und rührt 48 Stunden bei Raumtemperatur und anschließend 48 Stunden bei 75° C. Die Reaktionsmischung wird filtriert, das Filtrat wird eingeengt und der Rückstand in 100 ml Methylenchlorid aufgenommen, mit 30 ml 2N-Salzsäure und gesättigter Natriumhydrogencarbonatlösung gewaschen, getrocknet über Magnesiumsulfat und im Vakuum eingeengt.

Man erhält 3,0 g (55 % der Theorie) an 5-[1-(Ethoxycarbonyl-ethyloxycarbonyl]-4-cyano-1-(2,6-dichlor-4-trifluormethyl-phenyl)-pyrazol als ölige Substanz.

$^1$H-NMR ($CDCl_3$) $\delta$ = 8,13 ppm (3-Pyrazol H)

Beispiel 8:

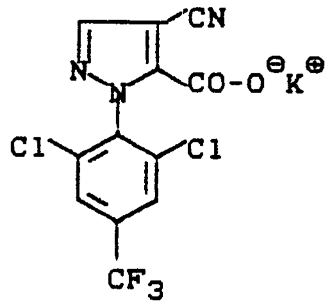

(Verfahren (b-β))

3,5 g (0,01 Mol) 5-Carboxy-4-cyano-1-(2,6-dichlor-4-trifluormethyl-phenyl)-pyrazol werden mit 0,1 N wässriger Kaliumhydroxidlösung titriert. Die wässrige Lösung versetzt man mit 50 ml Isopropanol und engt unter vermindertem Druck bis zur Trockene ein.

Man erhält 3,6 g (93 % der Theorie) des Kaliumsalzes des 5-Carboxy-4-cyano-1-(2,6-dichlor-4-trifluormethyl-phenyl)-pyrazols vom Schmelzpunkt 230° C (Zersetzung).

Beispiel 9:

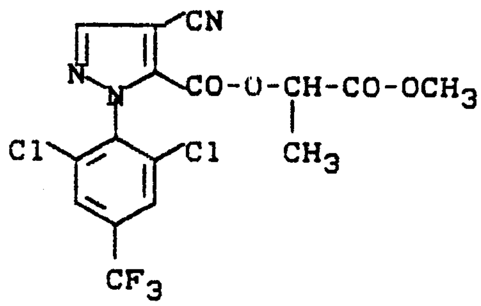

(Verfahren (b-γ))

3,5 g (0,01 Mol) 5-Carboxy-4-cyano-1-(2,6-dichlor-4-trifluormethyl-phenyl)-pyrazol werden in 50 ml Methylethylketon gelöst und mit 1,4 g (0,01 Mol) Kaliumcarbonat versetzt. Die Reaktionsmischung wird solange unter Rückfluß erhitzt, bis sich ein weißer Niederschlag gebildet hat. Danach läßt man auf Raumtemperatur abkühlen, tropft 2,0 g (0,012 Mol) α-Brompropionsäuremethylester zu und rührt 3 Stunden bei 75°C. Die Reaktionsmischung wird mit Ether verdünnt, filtriert und das Filtrat mit 2-prozentiger wässriger Salzsäure und gesättigter Natriumhydrogencarbonatlösung gewaschen, über Magnesiumsulfat getrocknet und im Vakuum eingeengt.

Man erhält 2,2 g (50 % der Theorie) an 5-[1-(Methoxycarbamoyl)-ethyloxycarbonyl]-4-cyano-1-(2,6-dichlor-4-trifluormethyl-phenyl)-pyrazol vom Schmelzpunkt 88° C.

Beispiel 10:

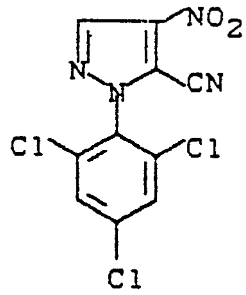

(Verfahren (c-β))

3,3 g (0,01 Mol) 5-Chlor-4-nitro-1-(2,4,6-trichlorphenyl)-pyrazol und 6,0 g (0,02 Mol) Tetrabutylammoniumcyanid werden in 100 ml Toluol 48 Stunden bei Raumtemperatur gerührt. Zur Aufarbeitung wäscht man mehrmals mit Wasser, trocknet über Natriumsulfat, engt im Vakuum ein und reinigt das ölige Rohprodukt chromatographisch (Kieselgel; Laufmittel: Chloroform/Aceton 9:1).

Man erhält 1,5 g (47 % der Theorie) an 5-Cyano-4-nitro-1-(2,4,6-trichlorphenyl)-pyrazol vom Schmelzpunkt 143° C.

In entsprechender Weise und gemäß den allgemeinen Angaben zur Herstellung erhält man die folgenden 1-Aryl-pyrazole der allgemeinen Formel (I):

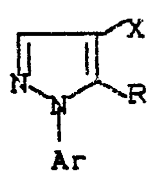

(I)

Tabelle 2

| Bsp. Nr. | X | R | Ar | Schmelz-punkt/° C |
|---|---|---|---|---|
| 11 | CN | -COOH | 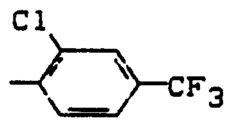 | 145-148 |
| 12 | CN | $-COO^{\ominus}\ Na^{\oplus}$ | 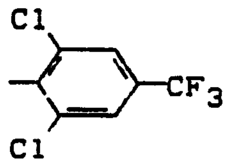 | 260-263 (Zers.) |
| 13 | CN | $-COO^{\ominus}H_3N^{\oplus}CH(CH_3)_2$ | 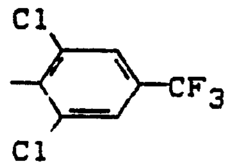 | 156-162 |
| 14 | CN | $-CO-N(CH_3)_2$ | 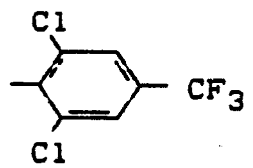 | 102-105 |
| 15 | CN | $-CO-NH-C_2H_5$ | 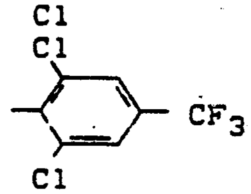 | 131-135 |
| 16 | CN | $-CO-NH-CH_3$ | 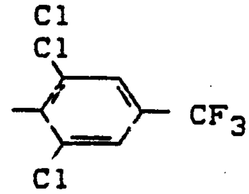 | 189-193 |
| 17 | CN | $-COOCH_3$ | 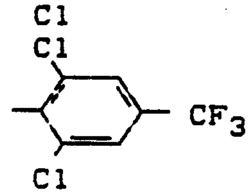 | 160-164 |
| 18 | CN | $-COOCH(CH_3)_2$ | 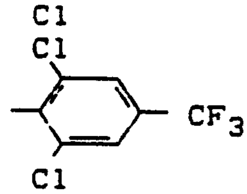 | 80-85 |
| 19 | $NO_2$ | $-COO^{\ominus}\ Na^{\oplus}$ | 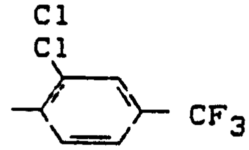 | 173 (Zers.) |

Tabelle 2 (Fortsetzung

| Bsp. Nr. | X | R | Ar | Schmelz-punkt/° C |
|---|---|---|---|---|
| 20 | $NO_2$ | $-COOC_2H_5-$ | 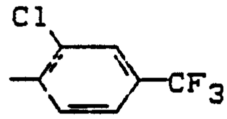 | Öl |
| 21 | $NO_2$ | $-COOCH_3$ | 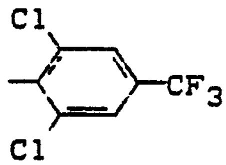 | 62-66 |
| 22 | $NO_2$ | $-COO(CH_2)_2CH_3$ | 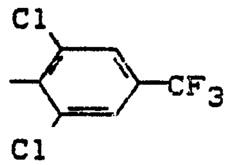 | 54-58 |
| 23 | $NO_2$ | $-COOCH(CH_3)_2$ | 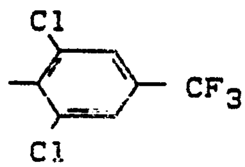 | Öl |
| 24 | $NO_2$ | $-CO-N(C_2H_5)_2$ | 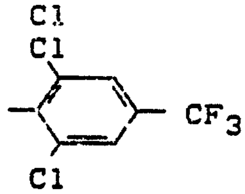 | 140 |
| 25 | $NO_2$ | $-CO-NH-C_2H_5$ | 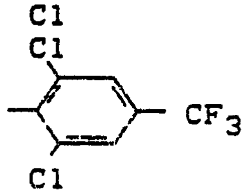 | 108-110 |
| 26 | $NO_2$ | $-CO-NH-(CH_2)_3-CH_3$ | 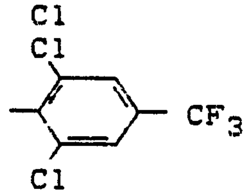 | 62-66 |
| 27 | $NO_2$ | $-COOC_2H_5-$ | 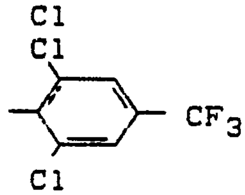 | Öl |
| 28 | CN | $-CO-O(CH_2)_2-OCH_3$ | 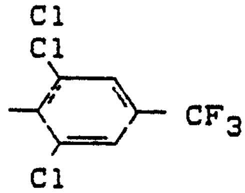 | 63-68 |

<u>Tabelle 2</u> (Fortsetzung)

| Bsp. Nr. | X | R | Ar | Schmelzpunkt/°C |
|---|---|---|---|---|
| 29 | CN | $-CO-SCH_3$ | 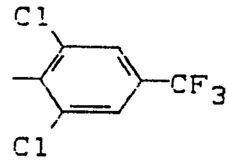 | 177-179 |
| 30 | $NO_2$ | $-CO-O-CH(CH_3)-COOC_2H_5$ | 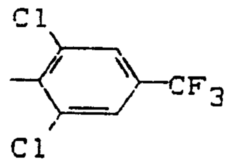 | Öl |
| 31 | $NO_2$ | $-COO^{\ominus}\ Na^{\oplus}$ | 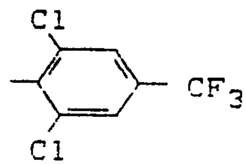 | Öl |
| 32 | $NO_2$ | $-CO-O-(CH_2)_2CH(CH_3)_2$ | 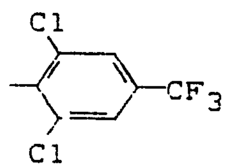 | Öl |
| 33 | $NO_2$ | -CO-O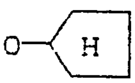 | 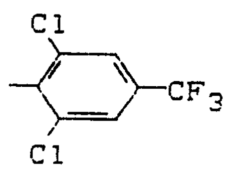 | 84-86 |
| 34 | $NO_2$ | $-CO-O-(CH_2)_2OCH_3$ | 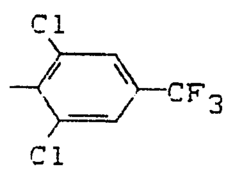 | Öl |
| 35 | $NO_2$ | $-CO-O-CH_2CH=CH_2$ | 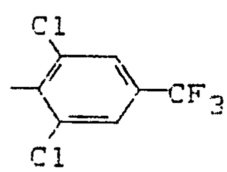 | Öl |
| 36 | $NO_2$ | $-CO-O-(CH_2)_2SCH_3$ | 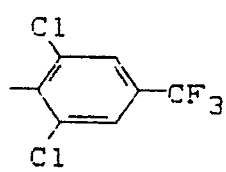 | Öl |

Tabelle 2 (Fortsetzung)

| Bsp. Nr. | X | R | Ar | Schmelzpunkt/° C |
|---|---|---|---|---|
| 37 | $NO_2$ | $-CO-O-CH_2C{\equiv}CH$ | 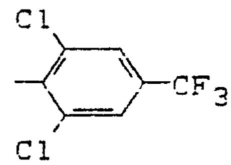 | 96-98 |
| 38 | $NO_2$ | $-CO-O-CH(CH_3)-COOC_2H_5$ | 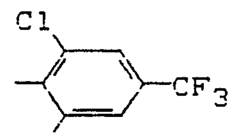 | Öl |
| 39 | $NO_2$ | -CN | 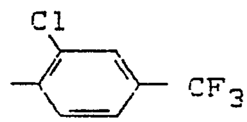 | 86 |
| 40 | $NO_2$ | $-CO-O-CH_2-COOC_4H_9$ | 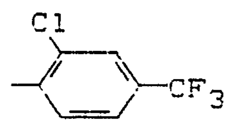 | Öl |

Herstellung der Ausgangsverbindungen

Beispiel II-1:

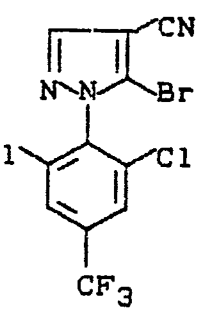

35 g (0,109 Mol) 5-Amino-4-cyano-1-(2,6-dichlor-4-trifluormethyl-phenyl)-pyrazol in 137 ml (1,54 Mol) Bromoform werden tropfenweise unter Rühren innerhalb von 10 Minuten mit 38 ml (0,32 Mol) t-Butylnitrit versetzt, wobei die Temperatur der Reaktionsmischung auf 75° C ansteigt. Nach beendeter Zugabe rührt man eine weitere Stunde bei Rückflußtemperatur, engt die Mischung im Vakuum ein und reinigt das zurückbleibende Öl säulenchromatographisch (Kieselgel; Laufmittel: Methylenchlorid).

Man erhält 29 g (69 % der Theorie) an 5-Brom-4-cyano-1-(2,6-dichlor-4-trifluormethyl-phenyl)-pyrazol vom Schmelzpunkt 92° C.

In entsprechender Weise und gemäß den allgemeinen Angaben zur Herstellung erhält man die folgenden 5-Halogen-1-aryl-pyrazole der allgemeinen Formel (II):

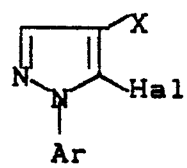

(II)

Tabelle 3

| Bsp. Nr. | X | Hal | Ar | Schmelzpunkt/° C |
|---|---|---|---|---|
| II-2 | CN | Br | 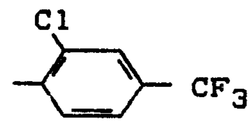 | 127 |
| II-3 | $NO_2$ | Br | 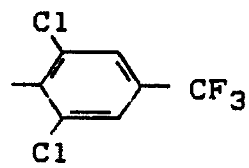 | 96 |

Beispiel III-1:

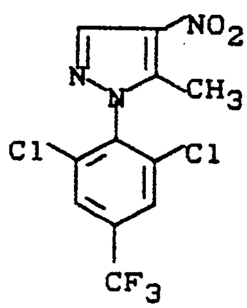

5 g (0,0147 Mol) 4-Carboxy-5-methyl-1-(2,6-dichlor-4-trifluormethyl-phenyl)-pyrazol werden in 36 ml konzentrierter Salzsäure gelöst. Man tropft 3,8 ml (0,0394 Mol) 98%ige Salpetersäure zu, erhitzt 1 Stunde auf 120° C und läßt dann abkühlen. Die Reaktionsmischung wird auf Eiswasser gegeben, mit Methylenchlorid extrahiert, die Methylenchloridphasen mit gesättigter Natriumhydrogencarbonatlösung gewaschen, über Magnesiumsulfat getrocknet und im Vakuum eingeengt.

Man erhält 3,7 g (74 % der Theorie) an 5-Methyl-4-nitro-1-(2,6-dichlor-4-trifluormethyl-phenyl)-pyrazol vom Schmelzpunkt 91-92° C.

Beispiel XIX-1:

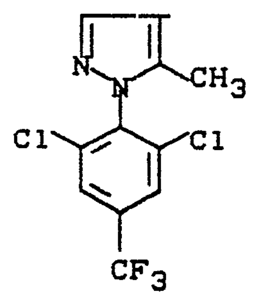

3,7 g (0,01 Mol) 4-Ethoxycarbonyl-5-methyl-1-(2,6-dichlor-4-trifluormethyl-phenyl)-pyrazol werden in 40 ml Bromwasserstoffsäure 48 Stunden auf 120-125° C erhitzt, wobei das entstehende Ethanol gleichzeitig abdestilliert wird. Anschließend wird die überschüssige Bromwasserstoffsäure abdestilliert, der Rückstand in Methylenchlorid aufgenommen und mit gesättigter Natriumhydrogencarbonatlösung gewaschen. Die organische Phase wird über Magnesiumsulfat getrocknet und im Vakuum eingeengt.

Man erhält 2,0 g (67 % der Theorie) an 5-Methyl-1-(2,6-dichlor-4-trifluormethyl-phenyl)-pyrazol vom Schmelzpunkt 78-84° C.

In entsprechender Weise erhält man

Beispiel XIX-2:

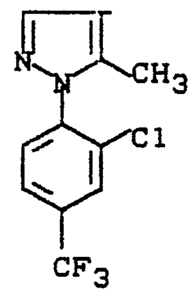

Schmelzpunkt 45-47° C.

Beispiel XVIIIa-1:

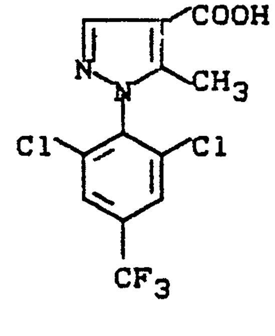

8,7 g (0,0237 Mol) 4-Ethoxycarbonyl-5-methyl-1-(2,6-dichlor-4-trifluormethyl-phenyl)-pyrazol werden in einer Lösung aus 50 ml Ethanol, 1,5 g (0,0375 Mol) Natriumhydroxid und 25 ml Wasser gelöst und 48 Stunden bei Raumtemperatur gerührt. Die Reaktionsmischung wird mit Methylenchlorid gewaschen und die alkalische Phase unter Eiskühlung mit 2N Salzsäure angesäuert. Anschließend wird mit Methylenchlorid extrahiert, die Methylenchloridphase über Magnesiumsulfat getrocknet und im Vakuum eingeengt.

Man erhält 8 g (100 % der Theorie) an 4-Carboxy-5-methyl-1-(2,6-dichlor-4-trifluormethyl-phenyl)-pyrazol vom Schmelzpunkt 187-189° C.

In entsprechender Weise erhält man

Beispiel XVIIIa-2:

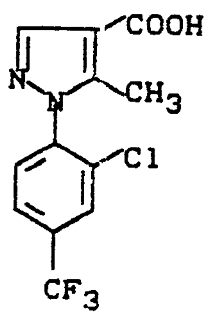

Schmelzpunkt 161 -162° C.

Beispiel XVIII-1:

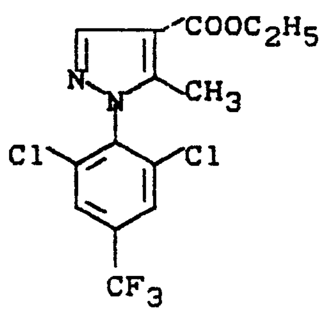

5 g (0,013 Mol) N-[2-Ethoxycarbonyl-2-acetyl-vinyl]-N'-(2,6-dichlor-4-trifluormethyl-phenyl)-hydrazin werden in 50 ml Ethanol gelöst, mit 1 ml konzentrierter Schwefelsäure versetzt und 1 Stunde unter Rückfluß erhitzt. Die Reaktionsmischung wird im Vakum eingeengt, in Methylenchlorid aufgenommen, mit gesättigter Natriumhydrogencarbonatlösung gewaschen, die Methylenchloridphase über Magnesiumsulfat getrocknet und im Vakuum eingeengt.

Man erhält 4,1 g (86 % der Theorie) an 4-Ethoxycarbonyl-4-methyl-1-(2,6-dichlor-4-trifluormethyl-phenyl)-pyrazol vom Schmelzpunkt 61 -62° C.

In entsprechender Weise erhält man

Beispiel XVIII-2

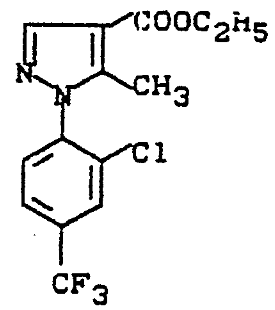

Schmelzpunkt 69 -70° C.

Beispiel XVII-1:

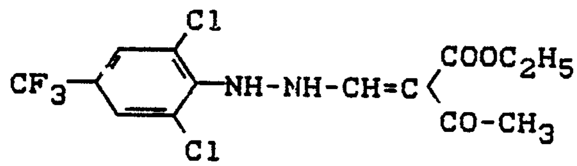

Zu einer Lösung aus 9,3 g (0,5 Mol) Ethoxymethylenacetessigsäureethylester in 50 ml Diethylether gibt man bei 0° C tropfenweise unter Rühren 12,25 g (0,05 Mol) 2,6-Dichlor-4-trifluormethylphenylhydrazin in 25 ml Diethylether. Nach beendeter Zugabe rührt man eine weitere Stunde bei Raumtemperatur und engt dann im Vakuum ein.

Man erhält 19 g (100 % der Theorie) an N-[2-Ethoxycarbonyl-2-acetyl-vinyl]-N'-(2,6-dichlor-4-trifluormethylphenyl)-hydrazin vom Schmelzpunkt 75-76° C.

Beispiel IV-1:

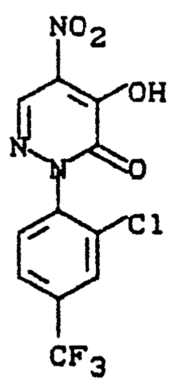

31 g (0,09 Mol) 4,5-Dichlor-1-(2-chlor-4-trifluormethylphenyl)-pyridazin-6-on werden in 100 ml Dimethylformamid gelöst. Zu dieser Mischung läßt man bei 105° C eine Lösung aus 31,7 g (0,46 Mol) Natriumnitrit in 32 ml Wasser tropfen und rührt nach beendeter Zugabe eine weitere Stunde bei Rückflußtemperatur. Nach dem Abkühlen wird mit Wasser versetzt, unter Eiskühlung mit 2N Salzsäure angesäuert und zweimal mit Methylenchlorid extrahiert. Die vereinigten organischen Phasen werden über Magnesiumsulfat getrocknet und im Vakuum eingeengt.

Durch Verreiben mit Ethanol erhält man 18,4 g (61 % der Theorie) an 5-Hydroxy-4-nitro-1-(2-Chlor-4-trifluormethyl-phenyl)-pyridazin-6-on vom Schmelzpunkt 213° C.

In entsprechender Weise erhält man Beispiel IV-2:

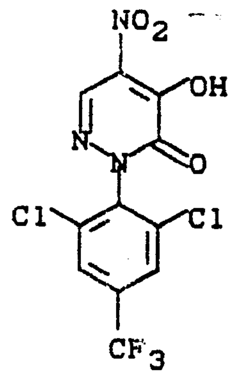

Schmelzpunkt 180° C (Zers.)

Beispiel XXII-1:

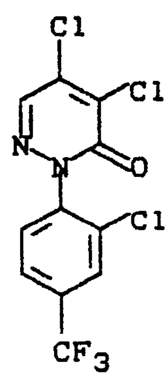

105 g (0,29 Mol) Mucochlorsäure-(2-Chlor-4-trifluormethyl-phenyl)-hydrazon werden in 500 ml Eisessig gelöst und 1 Stunde unter Rückfluß erhitzt. Die noch heiße Lösung wird mit etwas Wasser versetzt, um die Kristallisation zu beschleunigen. Der ausfallende Niederschlag wird abgesaugt und mit Petrolether verrieben.

Man erhält 72 g (72 % der Theorie) an 4,5-Dichlor-1-(2-chlor-4-trifluormethyl-phenyl)-pyridazin-6-on vom Schmelzpunkt 183-184° C.

In entsprechender Weise erhält man Beispiel XXII-2:

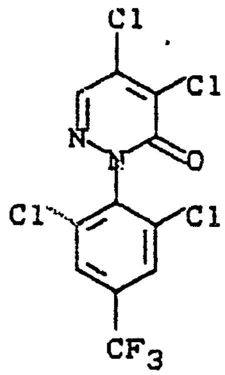

Schmelzpunkt 177° C

Beispiel XXI-1:

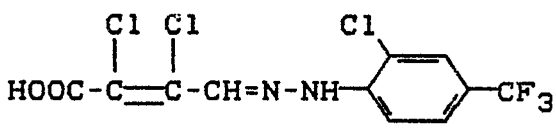

105,3 g (0,5 Mol) 2-Chlor-4-trifluormethylphenylhydrazin werden in 500 ml Toluol gelöst, bei Raumtemperatur mit 84,5 g (0,5 Mol) Mucochlorsäure versetzt und 2 Stunden bei Raumtemperatur nachgerührt.

Durch Absaugen und Trocknen des kristallinen Niederschlags erhält man 180 g (100 % der Theorie) an Mucochlorsäure-(2 chlor-4-trifluormethyl-phenyl)-hydrazon vom Schmelzpunkt 176° C.

In entsprechender Weise erhält man Beispiel XXI-2 :

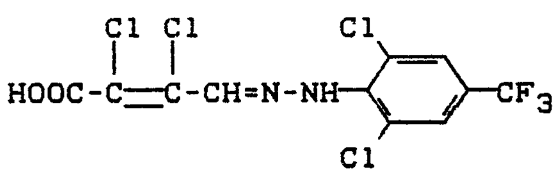

Schmelzpunkt 154-155° C

Anwendungsbeispiele:

In den folgenden Anwendungsbeispielen wurde die nachstehend aufgeführte Verbindung als Vergleichsubstanz eingesetzt:

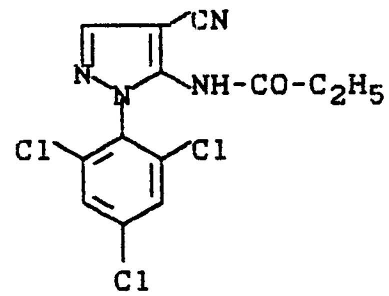

4-Cyano-5-propionamido-1-(2,4,6-trichlorphenyl)-pyrazol (bekannt aus DE-OS 3 226 513)

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Überlegenheit in der Nutzpflanzenselektivität, insbesondere bei Mais, gegenüber der Vergleichssubstanz zeigen in diesem Test z.B. die Verbindungen gemäß den Herstellungsbeispielen: 1, 3, 8 und 20

Beispiel B

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 -15 cm haben so, daß die jeweils gewünschten Wirkstoffmengen pro Flächeneinheit ausgebracht werden. Die Konzentration der Spritzbrühe wird so bewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Überlegenheit in der Nutzpflanzenselektivität, insbesondere bei Weizen und Gerste, gegenüber der Vergleichssubstanz (A) zeigen in diesem Test z.B. die Verbindungen gemäß den Herstellungsbeispielen 2, 20 und 21

## Ansprüche

1. 1-Aryl-pyrazole der allgemeinen Formel (I),

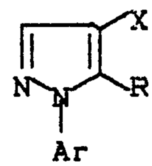

(I)

in welcher
X für Cyano oder Nitro steht,
R für Cyano oder für einen Rest -CO-Y-$R^1$ steht,
wobei
Y für Sauerstoff, Schwefel oder einen Rest $-\underset{R^2}{\underset{|}{N}}-$ steht,
$R^1$ für Wasserstoff, für Alkyl, Hydroxyalkyl, Alkoxyalkyl, Alkylthioalkyl, Halogenalkyl, Alkenyl, Halogenalkenyl, Alkinyl oder für jeweils gegebenenfalls substituiertes Cycloalkyl, Aralkyl oder Aryl steht, für einen Rest

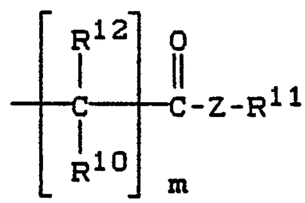

steht,
oder $R^1$ für den Fall, daß Y für Sauerstoff, Schwefel oder einen Rest $-\underset{|}{N}-SO_2-R^3$ steht, auch für ein salzartig gebundenes anorganisches oder organisches Kation steht,
$R^2$ für Wasserstoff, für Alkyl, Hydroxyalkyl, Alkoxyalkyl, Alkylthioalkyl, Halogenalkyl, Alkenyl, Alkinyl oder Halogenalkenyl, für jeweils gegebenenfalls substituiertes Cycloalkyl, Aralkyl oder Aryl steht oder für einen Rest $-SO_2-R^3$ steht, wobei
$R^3$ für jeweils gegebenenfalls substituiertes Alkyl Aralkyl oder Aryl steht,
$R^{10}$ und $R^{12}$ unabhängig voneinander für Wasserstoff oder Alkyl stehen,
$R^{11}$ für Wasserstoff, Alkyl, Alkenyl oder Alkinyl steht,
Z für Sauerstoff, Schwefel oder einen N-Alkyl-Rest steht und
m für eine Zahl 1, 2, 3, 4, 5 oder 6 steht,
und
Ar für einen Rest

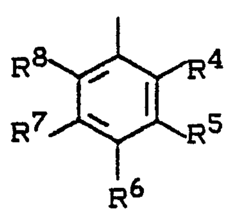

oder für einen Rest

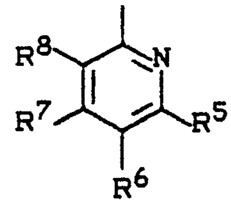

steht,
wobei
$R^4$ für Cyano, Nitro, Halogen, Alkyl, Alkoxy, Alkoxycarbonyl, Halogenalkyl, Halogenalkoxy, oder für einen Rest $-S(O)_n-R^9$ steht,
$R^5$, $R^6$, $R^7$ und $R^8$ unabhängig voneinander für Wasserstoff, Cyano, Nitro, Halogen, Alkyl, Alkoxy, Alkoxycarbonyl, Halogenalkyl, Halogenalkoxy oder für einen Rest $-S(O)_n-R^9$ stehen, wobei
$R^9$ für Alkyl, Halogenalkyl, Amino, Alkylamino oder Dialkylamino steht, und
n für eine Zahl 0, 1 oder 2 steht.

2. 1-Aryl-pyrazole der Formel (I) gemäß Anspruch 1, dadurch gekennzeichnet, daß
X für Cyano oder Nitro steht,
R für Cyano oder für einen Rest $-CO-Y-R^1$ steht,
wobei
Y für Sauerstoff, Schwefel oder für einen Rest - N -steht,
$R^2$
$R^1$ für Wasserstoff, für jeweils geradkettiges oder verzweigtes Alkyl, Hydroxyalkyl, Alkoxyalkyl, Alkylthioalkyl, Halogenalkyl, Alkenyl, Halogenalkenyl oder Alkinyl mit jeweils bis zu 12 Kohlenstoffatomen und im Falle des Halogenalkyl und des Halogenalkenyl mit jeweils 1 bis 15 gleichen oder verschiedenen Halogenatomen, für Cycloalkyl mit 3 bis 7 Kohlenstoffatomen oder für jeweils gegebenenfalls einfach oder mehrfach, gleich oder verschieden durch Halogen, Cyano, Nitro sowie jeweils geradkettiges oder verzweigtes Alkyl, Alkoxy, Alkylthio oder Halogenalkyl mit jeweils 1 bis 4 Kohlenstoffatomen und im Fall des Halogenalkyls mit 1 bis 9 gleichen oder verschiedenen Halogenatomen, substituiertes Phenyl, Benzyl oder Phenethyl steht, außerdem für einen Rest

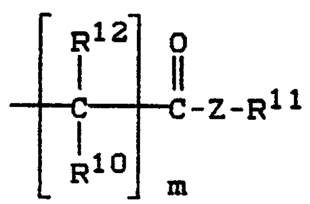

steht,
und außerdem $R^1$ für den Fall, daß Y für Sauerstoff, Schwefel oder einen Rest - N $-SO_2-R^3$ steht, auch für ein Äquivalent eines Alkali-, Erdalkali-, Kupfer-, Zink-, Mangan-, Zinn-, Eisen-, Cobalt-oder Nickelkations oder für ein gegebenenfalls einfach oder mehrfach, gleich oder verschieden substituiertes Ammonium-, Phosphonium-oder Sulfoniumkations steht, wobei als Substituenten jeweils in Frage kommen: geradkettiges oder verzweigtes Alkyl mit 1 bis 18 Kohlenstoffatomen, Phenyl oder Benzyl;
$R^2$ für Wasserstoff, für jeweils geradkettiges oder verzweigtes Alkyl, Hydroxyalkyl, Alkoxyalkyl, Alkylthioalkyl, Halogenalkyl, Alkenyl, Halogenalkenyl oder Alkinyl mit jeweils bis zu 12 Kohlenstoffatomen und im Falle des Halogenalkyl bzw. des Halogenalkenyl mit 1 bis 15 gleichen oder verschiedenen Halogenatomen, für Cycloalkyl mit 3 bis 7 Kohlenstoffatomen oder für jeweils gegebenenfalls einfach oder mehrfach, gleich oder verschieden durch Halogen, Cyano, Nitro sowie jeweils geradkettiges oder verzweigtes Alkyl, Alkoxy, Alkylthio oder Halogenalkyl mit jeweils 1 bis 4 Kohlenstoffatomen und im Fall des Halogenalkyls mit 1 bis 9 gleichen oder verschiedenen Halogenatomen substituiertes Phenyl, Benzyl oder Phenethyl steht, außerdem für einen Rest $-SO_2-R^3$ steht,
wobei
$R^3$ für jeweils geradkettiges oder verzweigtes Alkyl oder Halogenalkyl mit jeweils 1 bis 6 Kohlenstoffatomen und im Fall des Halogenalkyls mit 1 bis 9 gleichen oder verschiedenen Halogenatomen oder für gegebenenfalls einfach oder mehrfach, gleich oder verschieden substituiertes Phenyl, Benzyl oder Phenylethyl

steht, wobei als Substituenten jeweils in Frage kommen: Halogen, Cyano, Nitro, jeweils geradkettiges oder verzweigtes Alkyl, Alkoxy, Alkylthio oder Halogenalkyl mit jeweils 1 bis 4 Kohlenstoffatomen und im Fall des Halogenalkyl mit 1 bis 9 gleichen oder verschiedenen Halogenatomen; und

$R^{10}$ und $R^{12}$ unabhängig voneinander jeweils für Wasserstoff oder für geradkettiges oder verzweigtes Alkyl mit 1 bis 4 Kohlenstoffatomen stehen,

$R^{11}$ für Wasserstoff oder für jeweils geradkettiges oder verzweigtes Alkyl, Alkenyl oder Alkinyl mit jeweils bis zu 6 Kohlenstoffatomen steht,

Z für Sauerstoff, Schwefel oder einen N-$C_1$-$C_4$-Alkyl-Rest steht und

m für einen Zahl 1, 2, 3, 4, 5 oder 6 steht,

Ar für einen Rest

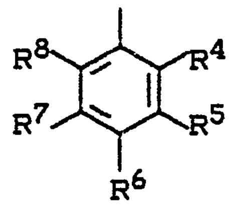

oder für einen Rest

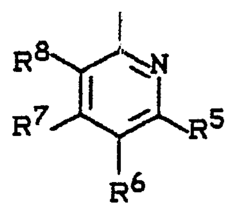

steht,

wobei

$R^4$ für Cyano, Nitro, Halogen für jeweils geradkettiges oder verzweigtes Alkyl, Alkoxy, Alkoxycarbonyl mit jeweils 1 bis 4 Kohlenstoffatomen, für jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen oder für einen Rest -$S(O)_n$-$R^9$ steht,

$R^5$, $R^6$, $R^7$ und $R^8$ unabhängig voneinander für Wasserstoff, Cyano, Nitro, Halogen, für jeweils geradkettiges oder verzweigtes Alkyl, Alkoxy oder Alkoxycarbonyl mit jeweils 1 bis 4 Kohlenstoffatomen, für jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen oder für einen Rest -$S(O)_n$-$R^9$ stehen, wobei

$R^9$ für Amino, für jeweils geradkettiges oder verzweigtes Alkyl, Alkylamino, Dialkylamino oder Halogenalkyl mit jeweils 1 bis 4 Kohlenstoffatomen in den einzelnen Alkylteilen und im Fall des Halogenalkyl mit 1 bis 9 gleichen oder verschiedenen Halogenatomen steht,

n für eine Zahl 0, 1 oder 2 steht.

3. 1-Aryl-pyrazole der Formel (I) gemäß Anspruch 1, dadurch gekennzeichnet, daß

X für Cyano oder Nitro steht,

R für Cyano oder für einen Rest -CO-Y-R steht, wobei

Y für Sauerstoff, Schwefel oder einen Rest $-\underset{R^2}{\underset{|}{N}}-$ steht,

$R^1$ für Wasserstoff, für gebenenfalls ein-bis dreifach, gleich oder verschieden durch Fluor, Chlor, Hydroxy, Methoxy, Ethoxy, Methylthio oder Ethylthio substituiertes Methyl, Ethyl, n-oder i-Propyl, n-, i-, s-oder t-Butyl, n-oder i-Pentyl, für gegebenenfalls ein-bis dreifach, gleich oder verschieden durch Fluor oder Chlor substituiertes Allyl, Propenyl oder Butenyl, für Propargyl, Propinyl, Butinyl oder Pentinyl, für Cyclopropyl, Cylcopentyl, Cyclohexyl, für jeweils gegebenenfalls ein-bis fünffach, gleich oder verschieden durch Fluor, Chlor, Brom, Cyano, Nitro, Methyl, Ethyl, Methoxy, Ethoxy, Methylthio oder Trifluormethyl subsituiertes Benzyl oder Phenyl steht, außerdem für einen Rest

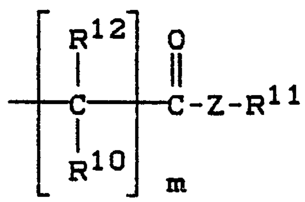

steht,

und darüberhinaus für den Fall, daß Y für Sauerstoff, Schwefel oder einen Rest $-\underset{|}{N}-SO_2-R^3$ steht,

auch für ein Äquivalent eines Natrium-, Kalium-, Magnesium-, Calcium-, Barium-, Kupfer-, Zink-, Mangan-, Zinn-, Eisen-, Cobalt-oder Nickelkations oder für ein gegebenenfalls ein-bis dreifach, gleich oder verschieden durch Methyl, Ethyl, n-oder i-Propyl, n-, i-, s-oder t-Butyl, Benzyl oder Phenyl substituiertes Ammonium-, Phosphonium oder Sulfoniumkation steht,

$R^2$ für Wasserstoff, für gebenenfalls ein-bis dreifach, gleich oder verschieden durch Fluor, Chlor, Hydroxy, Methoxy, Ethoxy, Methylthio oder Ethylthio substituiertes Methyl, Ethyl, n-oder i-Propyl, n-, i-, s-oder t-Butyl, n-oder i-Pentyl, für gegebenenfalls ein-bis dreifach, gleich oder verschieden durch Fluor oder Chlor substituiertes Allyl, Propenyl oder Butenyl, für Propargyl, Propinyl, Butinyl oder Pentinyl, für Cyclopropyl, Cyclopentyl, Cyclohexyl, für jeweils gegebenenfalls ein-bis fünffach, gleich oder verschieden durch Fluor, Chlor, Brom, Cyano, Nitro, Methyl, Ethyl, Methoxy, Ethoxy, Methylthio oder Trifluormethyl substituiertes Benzyl oder Phenyl steht, und außerdem für einen Rest

$-SO_2-R^3$ steht, wobei

$R^3$ für Methyl, Ethyl, n-oder i-Propyl, n-, i-, s-oder t-Butyl oder für gegebenenfalls ein-bis fünffach, gleich oder verschieden durch Fluor, Chlor, Brom, Cyano, Nitro, Methyl, Ethyl, Methoxy, Methylthio oder Trifluormethyl substituiertes Benzyl oder Phenyl steht,

$R^{10}$ und $R^{12}$ unabhängig voneinander jeweils für Wasserstoff, Methyl, Ethyl, n-oder i-Propyl stehen,

$R^{11}$ für Wasserstoff, Methyl, Ethyl, n-oder i-Propyl, n-, i-, s-oder t-Butyl, für Allyl, Propenyl, Butenyl, Propargyl, Propinyl oder Butinyl steht,

Z für Sauerstoff, Schwefel, einen N-Methylrest oder einen N-Ethylrest steht und

m für eine Zahl 1, 2, 3 oder 4 steht,

Ar für einen Rest

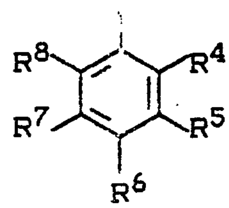

oder für einen Rest

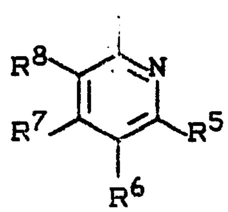

steht,

wobei

$R^4$ für Cyano, Nitro, Fluor, Chlor, Brom, Methyl, Ethyl, n-oder i-Propyl, Methoxy, Ethoxy, Trifluormethyl, Trichlormethyl, Dichlorfluormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, für Trifluormethoxy, Trichlormethoxy, Dichlor fluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder für einen Rest $-S(O)_n-R^9$ steht und

$R^5$, $R^6$, $R^7$ und $R^8$ unabhängig voneinander jeweils für Wasserstoff, Cyano, Nitro, Fluor, Chlor, Brom, Methyl, Ethyl, n-oder i-Propyl, n-, i-, s-oder t-Butyl, Methoxy, Ethoxy, Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, für Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder für einen Rest $-S(O)_n-R^9$ stehen, wobei

$R^9$ für Amino, Methylamino, Ethylamino, Dimethylamino, Diethylamino, Fluordichlormethyl, Difluormethyl, Tetrafluorethyl, Trifluorchlorethyl, Trifluormethyl, Methyl oder Ethyl steht,

n für eine Zahl 0, 1 oder 2 steht.

4. Verfahren zur Herstellung von 1-Aryl-pyrazolen der Formel (I),

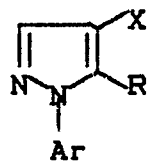

(I)

in welcher

X für Cyano oder Nitro steht,

R für Cyano oder für einen Rest $-CO-Y-R^1$ steht,

wobei

Y für Sauerstoff, Schwefel oder einen Rest $-\underset{R^2}{N}-$ steht,

$R^1$ für Wasserstoff, für Alkyl, Hydroxyalkyl, Alkoxyalkyl, Alkylthioalkyl, Halogenalkyl, Alkenyl, Halogenalkenyl, Alkinyl oder für jeweils gegebenenfalls substituiertes Cycloalkyl, Aralkyl oder Aryl steht, für einen Rest

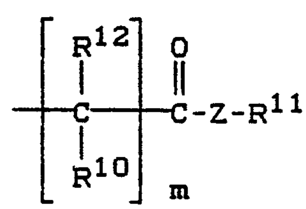

steht,

oder $R^1$ für den Fall, daß Y für Sauerstoff, Schwefel oder einen Rest $-\underset{|}{N}-SO_2-R^3$ steht, auch für ein salzartig gebundenes anorganisches oder organisches Kation steht,

$R^2$ für Wasserstoff, für Alkyl, Hydroxyalkyl, Alkoxyalkyl, Alkylthioalkyl, Halogenalkyl, Alkenyl, Alkinyl oder Halogenalkenyl, für jeweils gegebenenfalls substituiertes Cycloalkyl, Aralkyl oder Aryl steht oder für einen Rest $-SO_2-R^3$ steht, wobei

$R^3$ für jeweils gegebenenfalls substituiertes Alkyl, Aralkyl oder Aryl steht,

$R^{10}$ und $R^{12}$ unabhängig voneinander für Wasserstoff oder Alkyl stehen,

$R^{11}$ für Wasserstoff, Alkyl, Alkenyl oder Alkinyl steht,

Z für Sauerstoff, Schwefel oder einen N-Alkyl-Rest steht und

m für eine Zahl 1, 2, 3, 4, 5 oder 6 steht,

und

Ar für einen Rest

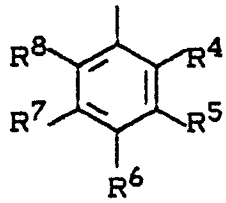

oder für einen Rest

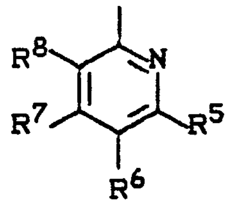

steht,
wobei
$R^4$ für Cyano, Nitro, Halogen, Alkyl, Alkoxy, Alkoxycarbonyl, Halogenalkyl, Halogenalkoxy oder für einen Rest $-S(O)_n-R^9$ steht,
$R^5$, $R^6$, $R^7$ und $R^8$ unabhängig voneinander für Wasserstoff, Cyano, Nitro, Halogen, Alkyl, Alkoxy, Alkoxycarbonyl, Halogenalkyl, Halogenalkoxy oder für einen Rest $-S(O)_n-R^9$ stehen, wobei
$R^9$ für Alkyl, Halogenalkyl, Amino, Alkylamino oder Dialkylamino steht und
n für eine Zahl 0, 1 oder 2 steht,
dadurch gekennzeichnet, daß man
(a) 5-Carboxy-1-aryl-pyrazole der allgemeinen Formel (Ia),

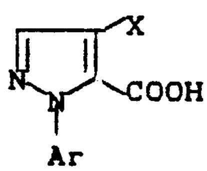
(Ia)

in welcher
X und Ar die oben angegebenen Bedeutungen haben,
erhält, wenn man
(a-α) 5-Halogen-1-aryl-pyrazole der Formel (II)

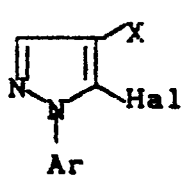
(II)

in welcher
X und Ar die oben angegebenen Bedeutungen haben und
Hal für Halogen steht,
mit Kohlendioxid in Gegenwart einer Lithium-organischen Verbindung und in Gegenwart eines Verdünnungsmittels umsetzt
oder daß man 5-Carboxy-4-nitro-1-aryl-pyrazole der Formel (Ia-1)

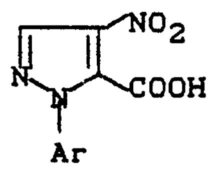

(Ia-1)

in welcher
Ar die oben angegebene Bedeutung hat,
erhält, wenn man
(a-β) 5-Methyl-4-nitro-1-aryl-pyrazole der Formel (III)

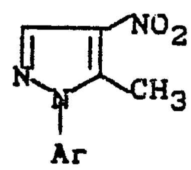

(III)

in welcher
Ar die oben angegebene Bedeutung hat,
mit Kaliumpermanganat in Gegenwart von Luftsauerstoff und in Gegenwart eines Verdünnungsmittels oxidiert,
oder wenn man
(a-γ) 5-Hydroxy-4-nitro-1-aryl-pyridazin-6-one der Formel (IV),

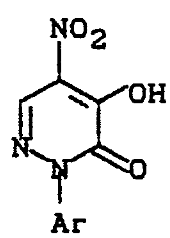

(IV)

in welcher
Ar die oben angegebene Bedeutung hat,
mit Basen in Gegenwart eines Verdünnungsmittels umsetzt,
oder daß man
(b) 5-Carboxy-1-aryl-pyrazol-Derivate der Formel (Ib-1),

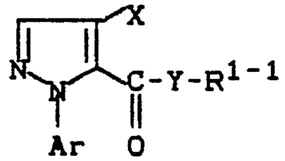

(Ib-1)

in welcher
X, Y und Ar die oben angegebene Bedeutung haben und
$R^{1-1}$ für Alkyl, Hydroxyalkyl, Alkoxyalkyl, Alkylthioalkyl, Halogenalkyl, Alkenyl, Halogenalkenyl, Alkinyl oder für jeweils gegebenenfalls substituiertes Cycloalkyl, Aralkyl oder Aryl steht, für einen Rest

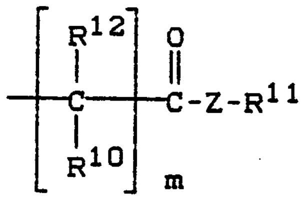

steht,
und außerdem $R^{1-1}$ für den Fall, daß Y für Schwefel oder einen Rest -N(\)-$R^2$ steht, auch für Wasserstoff steht,

wobei
$R^{10}$ und $R^{12}$ unabhängig voneinander für Wasserstoff oder Alkyl stehen,
$R^{11}$ für Wasserstoff, Alkyl, Alkenyl oder Alkinyl steht,
Z für Sauerstoff, Schwefel oder einen N-Alkyl-Rest steht und
m für eine Zahl 1, 2, 3, 4, 5 oder 6 steht,
erhält, wenn man
(b-α) 5-Carboxy-1-aryl-pyrazole der Formel (Ia)

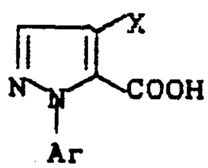
(Ia)

in welcher
X und Ar die oben angegebene Bedeutung haben,
mit Alkoholen, Aminen oder Thiolen der Formel (V)
$R^{1-1}$-Y-H (V)
in welcher
$R^{1-1}$ und Y die oben angegebenen Bedeutung haben,
gegebenenfalls in Gegenwart eines Reaktionshilfsmittels und gegebenenfalls in Gegenwart eines Verdünnungsmittels sowie gegebenenfalls in Gegenwart eine Säurebindemittels umsetzt;
oder daß man Salze der Formel (Ib-2)

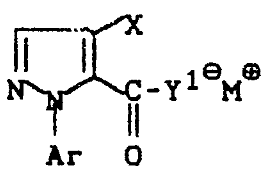
(Ib-2)

in welcher
X und Ar die oben angegebene Bedeutung haben,
$Y^1$ für Sauerstoff, Schwefel oder einen Rest -N-$SO_2$-$R^3$ steht und
$M^{\oplus}$ für ein anorganisches oder organisches Kation steht,
erhält, wenn man
(b-β) 5-Carboxy-1-aryl-Pyrazol-Derivate der Formel (Id)

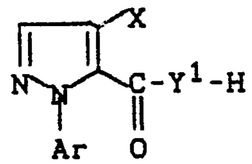

(Id)

in welcher
X, $Y^1$ und Ar die oben angegebene Bedeutung haben,
mit anorganischen oder organischen Basen gegebenenfalls in Gegenwart eines Verdünnungsmittels in üblicher Art und Weise umsetzt,
oder daß man 1-Aryl-pyrazole der Formel (Ib-3),

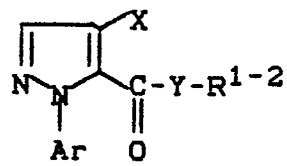

(Ib-3)

in welcher
X, Y und Ar die oben angegebene Bedeutung haben und
$R^{1-2}$ für Alkyl, Hydroxyalkyl, Alkoxyalkyl, Alkylthioalkyl, Halogenalkyl, Alkenyl, Halogenalkenyl, Alkinyl oder für jeweils gegebenenfalls substituiertes Cycloalkyl, Aralkyl, oder Aryl steht, außerdem für einen Rest

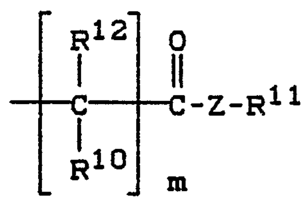

steht,
wobei
$R^{10}$ und $R^{12}$ unabhängig voneinander für Wasserstoff oder Alkyl stehen,
$R^{11}$ für Wasserstoff, Alkyl, Alkenyl oder Alkinyl steht,
Z für Sauerstoff, Schwefel oder einen N-Alkyl-Rest steht und
m für eine Zahl 1, 2, 3, 4, 5 oder 6 steht,
erhält, wenn man
(b-γ) Salze der Formel (Ib-2)

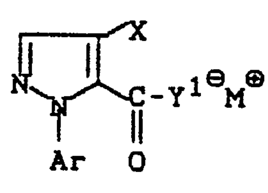

(Ib-2)

in welcher
X, $Y^1$, $M^+$ und Ar die oben angegebene Bedeutung haben,
mit Alkylierungsmitteln der Formel (VI)
$R^{1-2}$-E (VI)
in welcher
$R^{1-2}$ die oben angegebene Bedeutung hat und

E für eine elektronenanziehende Abgangsgruppe steht, gegebenenfalls in Gegenwart eines Verdünnungsmittels umsetzt, oder daß man

(c) 5-Cyano-1-aryl-pyrazole der Formel (Ic),

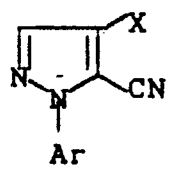

(Ic)

in welcher X und Ar die oben angegebene Bedeutung haben, erhält, wenn man

(c-α) 5-Carbonamid-1-aryl-pyrazole der Formel (Ib-4),

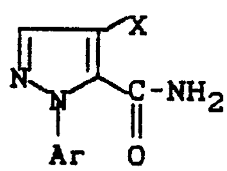

(Ib-4)

in welcher X und Ar die oben angegebene Bedeutung haben, dehydratisiert, oder wenn man

(c-β)5-Halogen-1-aryl-pyrazole der Formel (II)

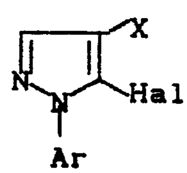

(II)

in welcher X und Ar die oben angegebene Bedeutung haben und Hal für Halogen steht, mit Cyaniden der Formel (VII)

$G^{\oplus}$-$CN^{\ominus}$ (VII)

in welcher $G^{\oplus}$ für ein geeignetes Gegenion steht, gegebenenfalls in Gegenwart eines Verdünnungsmittels und gegebenenfalls in Gegenwart eines Phasentransferkatalysators umsetzt.

5. Herbizide Mittel, gekennzeichnet durch einen Gehalt an mindestens einem 1-Aryl-pyrazol der Formel (I) gemäß den Ansprüchen 1 bis 4.

6. Verfahren zur Bekämpfung von Unkräutern, dadurch gekennzeichnet, daß man 1-Aryl-pyrazole der Formel (I) gemäß den Ansprüchen 1 bis 4 auf Unkräuter und/oder deren Lebensraum einwirken läßt.

7. Verwendung von 1-Aryl-pyrazolen der Formel (I) gemäß den Ansprüchen 1 bis 4 zur Bekämpfung von Unkräutern.

8. Verfahren zur Herstellung von herbiziden Mitteln, dadurch gekennzeichnet, daß man 1-Aryl-pyrazole der Formel (I) gemäß den Ansprüchen 1 bis 4 mit Streckmitteln und/oder oberflächenaktiven Substanzen vermischt.